(19) **Europäisches Patentamt**
**European Patent Office**
**Office européen des brevets**

(11) **EP 3 034 004 A1**

(12) **EUROPEAN PATENT APPLICATION**
published in accordance with Art. 153(4) EPC

(43) Date of publication:
**22.06.2016 Bulletin 2016/25**

(51) Int Cl.:
**A61B 8/00** (2006.01)

(21) Application number: **14836286.6**

(22) Date of filing: **07.08.2014**

(86) International application number:
**PCT/KR2014/007326**

(87) International publication number:
**WO 2015/023081 (19.02.2015 Gazette 2015/07)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(30) Priority: **12.08.2013 US 201361864799 P**

(71) Applicant: **Samsung Electronics Co., Ltd.**
**Gyeonggi-do 16677 (KR)**

(72) Inventors:
• **LEE, Min-gu**
**Hwaseong-si**
**Gyeonggi-do 445-730 (KR)**

• **SHIM, Hwan**
**Yongin-si**
**Gyeonggi-do 446-882 (KR)**
• **JUNG, Yun-sub**
**Hanam-si**
**Gyeonggi-do 465-815 (KR)**
• **CHEON, Byeong-geun**
**Anyang-si**
**Gyeonggi-do 431-852 (KR)**
• **KIM, Young-tae**
**Seongnam-si**
**Gyeonggi-do 463-956 (KR)**

(74) Representative: **Grootscholten, Johannes A.M. et al**
**Arnold & Siedsma**
**Bezuidenhoutseweg 57**
**2594 AC The Hague (NL)**

(54) **METHOD FOR PRODUCING ELASTIC IMAGE AND ULTRASONIC DIAGNOSTIC APPARATUS**

(57)     A method of generating an elastography image includes transmitting unfocused push signals comprising a plurality of ultrasound signals to an object in different directions; transmitting a detection ultrasound signal to the object in which a shear wave is generated by the unfocused push signals; receiving a response signal in response to the detection ultrasound signal from the object; and generating the elastography image of the object based on the response signal.

FIG. 6

START

TRANSMIT UNFOCUSED PUSH SIGNALS INCLUDING PLURALITY OF ULTRASOUND SIGNALS TO OBJECT IN DIFFERENT DIRECTIONS — S610

TRANSMIT DETECTION ULTRASOUND SIGNAL TO OBJECT IN WHICH SHEAR WAVE IS GENERATED BY UNFOCUSED PUSH SIGNALS — S620

RECEIVE RESPONSE SIGNAL TO DETECTION ULTRASOUND SIGNAL FROM OBJECT — S630

GENERATE ELASTOGRAPHY IMAGE OF OBJECT BASED ON RESPONSE SIGNAL — S640

END

EP 3 034 004 A1

**Description**

BACKGROUND

1. Field

The present disclosure relates to methods of generating an elastography image and ultrasound diagnosis apparatuses, and more particularly to, methods of generating a shear wave inside an object and acquiring a propagation characteristic of the shear wave so as to generate an elastography image and ultrasound diagnosis apparatuses for realizing the methods.

2. Description of the Related Art

Ultrasound diagnostic apparatuses transfer an ultrasound signal from a surface of an object to a certain part of a human body, and obtain a tomography image of a soft tissue or an image of blood flow by using information of an ultrasound signal reflected from an internal tissue of the human body.

Ultrasound diagnostic apparatuses are small and inexpensive, and display an acquired image in real time. Also, ultrasound diagnostic apparatuses have high stability because an object is not exposed to X-rays or the like, and thus are widely used along with other image diagnostic apparatuses, such as X-ray diagnostic apparatuses, computerized tomography (CT) scanners, magnetic resonance imaging (MRI) apparatuses, and nuclear medicine diagnostic apparatuses.

An elastography method is one of ultrasound imaging modalities that display the elasticity of an object as an image. The elasticity of the object provides qualitative or quantitative information about diseases relating to elasticity of a body tissue, such as cancer, cirrhosis, etc.

Tumors are generally harder than normal tissue. That is, the elasticity of a tumor is less than that of normal tissue, and thus, when the same pressure is applied to the tumor and the normal tissue, a strain of the normal tissue is greater than that of the tumor. Therefore, the elastography method may be used to diagnose tumors or cancer. An ultrasound diagnosis apparatus may be very conveniently and quickly used compared to other medical image diagnosis apparatuses, and thus the elastography method using ultrasound waves in an initial diagnosis of diseases are very important.

A technology of using the propagation of a shear wave or a transversal wave among elastography technologies using ultrasound waves has been recently developed and spotlighted. In more detail, a technology of digitizing and imaging the elasticity of the object by transmitting an ultrasound signal to the object, applying an acoustic radiation force, and acquiring the propagation characteristic of the shear wave has been recently developed.

Examples of methods of generating the shear wave using ultrasound waves include a method of generating a spherical wave by transmitting an ultrasound signal focused on a point inside an object, a method of generating a plane wave in a diagonal direction by sequentially transmitting ultrasound signals focused on a plurality of points inside the object, and a method of generating a plane wave traveling in a direction perpendicular to a direction in which an ultrasound signal is irradiated by transmitting an unfocused ultrasound signal.

A method of generating a strong shear wave in a wide area inside the object is required to generate an exact elastography image.

Meanwhile, various medical imaging methods such as ultrasound imaging, MRI, etc. may be used to acquire a propagation characteristic of the generated shear wave inside the object.

When ultrasound waves are used, a method of transmitting an ultrasound signal having a sufficiently short repetition cycle within an area of interest, receiving a response signal to the transmitted ultrasound signal, and acquiring the propagation characteristic of the shear wave through signal processing of the response signal may be used to observe how the shear wave travels.

When ultrasound waves are used to acquire the propagation characteristic of the shear wave, a frame rate of the ultrasound signal may be greater than 1 kHz in consideration of a frequency and bandwidth of the shear wave. Thus, the propagation characteristic of the shear wave may be acquired by using a plane ultrasound wave having a high pulse repetition frequency (PRF) greater than 1 kHz.

However, since a scan line based general ultrasound diagnosis apparatus has a low frame rate, it is impossible to acquire the propagation characteristic of the shear wave by using the plane ultrasound wave. Thus, a technology of generating an elastography image based on the shear wave by using an ultrasound diagnosis apparatus having the low frame rate is required.

## EP 3 034 004 A1

SUMMARY

Provided are methods and apparatuses for generating an ultrasound elastography image, whereby a strong shear wave is generated with respect to a wide area inside an object so as to generate an exact elastography image.

Provided are methods and apparatuses for generating an ultrasound elastography image, whereby a propagation characteristic of a shear wave is acquired by using a low frame rate ultrasound signal.

Additional aspects will be set forth in part in the description which follows and, in part, will be apparent from the description, or may be learned by practice of the presented exemplary embodiments.

According to an aspect of an exemplary embodiment, a method of generating an elastography image includes transmitting unfocused push signals including a plurality of ultrasound signals to an object in different directions; transmitting a detection ultrasound signal to the object in which a shear wave is generated by the unfocused push signals; receiving a response signal in response to the detection ultrasound signal from the object; and generating the elastography image of the object based on the response signal.

The unfocused push signals may include the plurality of ultrasound signals transmitted to a plurality of focal points inside the object and generate the shear wave inside the object by an acoustic radiation force generated at the plurality of focal points.

The transmitting of the unfocused push signals may include transmitting the unfocused push signals to the object in different directions such that the unfocused push signals simultaneously arrive at a predetermined axis.

The unfocused push signals may be transmitted in different directions so that constructive interference occurs between the unfocused push signals on the predetermined axis, and the predetermined axis may be parallel to an axial direction of a plurality of transducer elements that transmit the unfocused push signals.

The transmitting of the unfocused push signals may include transmitting a first unfocused push signal transmitted from a first group of adjacent transducer elements included in a plurality of transducer elements; and transmitting a second unfocused push signal transmitted from a second group of adjacent transducer elements included in the plurality of transducer elements, and the first unfocused push signal and the second unfocused push signal are transmitted in different directions such that the first unfocused push signal and the second unfocused push signal are symmetrical to each other with respect to a predetermined axis.

A plurality of transducer elements that transmit the unfocused push signals may be classified into a first group and a second group including a same number of adjacent transducer elements with respect to a center of an array in which the plurality of transducer elements are arranged, the unfocused push signals include a first unfocused push signal transmitted from the first group and a second unfocused push signal transmitted from the second group, and the first unfocused push signal and the second unfocused push signal are transmitted in different directions such that the first unfocused push signal and the second unfocused push signal are symmetrical to each other with respect to a predetermined axis, and the predetermined axis is parallel to an axial direction of the plurality of transducer elements at the center of the array.

The transmitting of the unfocused push signals may include transmitting the first unfocused push signal in a direction inclined by $+\theta°$ with respect to the predetermined axis and transmits the second unfocused push signal in a direction inclined by $-\theta°$ with respect to the predetermined axis.

The unfocused push signals may include the plurality of ultrasound signals transmitted from the respective plurality of transducer elements, and the plurality of transducer elements may transmit the plurality of ultrasound signals whose intensity is reduced from a transducer element located at a center of an array in which the plurality of transducer elements are arranged to a transducer element located at an end of the array.

According to another aspect of an exemplary embodiment, a ultrasound diagnosis apparatus includes a transmission unit configured to transmit unfocused push signals including a plurality of ultrasound signals to an object in different directions and to transmit a detection ultrasound signal to the object in which a shear wave is generated by the unfocused push signals; an ultrasound transmission/reception unit including a reception unit configured to receive a response signal in response to the detection ultrasound signal from the object; and an image processing unit configured to generate an elastography image of the object based on the response signal.

The unfocused push signals may include the plurality of ultrasound signals transmitted to a plurality of focal points inside the object and generate the shear wave inside the object by an acoustic radiation force generated at the plurality of focal points.

The transmission unit may transmit the unfocused push signals to the object in different directions such that the unfocused push signals simultaneously arrive at a predetermined axis.

The unfocused push signals may be transmitted in different directions so that constructive interference occurs between the unfocused push signals on the predetermined axis, and the predetermined axis may be parallel to an axial direction of a plurality of transducer elements that transmit the unfocused push signals.

The transmission unit may transmit a first unfocused push signal transmitted from a first group of adjacent transducer elements included in a plurality of transducer elements and a second unfocused push signal transmitted from a second group of adjacent transducer elements included in the plurality of transducer elements, and the first unfocused push signal and the second unfocused push signal are transmitted in different directions such that the first unfocused push signal and the second unfocused push signal are symmetrical to each other with respect to a predetermined axis.

A plurality of transducer elements that transmit the unfocused push signals may be classified into a first group and a second group including a same number of adjacent transducer elements with respect to a center of an array in which the plurality of transducer elements are arranged, the unfocused push signals include a first unfocused push signal transmitted from the first group and a second unfocused push signal transmitted from the second group, and the first unfocused push signal and the second unfocused push signal are transmitted in different directions such that the first unfocused push signal and the second unfocused push signal are symmetrical to each other with respect to a predetermined axis, and the predetermined axis is parallel to an axial direction of the plurality of transducer elements at the center of the array.

The transmission unit may transmit the first unfocused push signal in a direction inclined by $+\theta°$ with respect to the predetermined axis and transmits the second unfocused push signal in a direction inclined by $-\theta°$ with respect to the predetermined axis.

The unfocused push signals may include the plurality of ultrasound signals transmitted from the respective plurality of transducer elements, and the plurality of transducer elements may transmit the plurality of ultrasound signals whose intensity is reduced from a transducer element located at a center of an array in which the plurality of transducer elements are arranged to a transducer element located at an end of the array.

According to another aspect of an exemplary embodiment, a method of generating an elastography image includes transmitting a first push signal including one or more ultrasound signals to an object; transmitting a second push signal including one or more ultrasound signals to the object based on information regarding propagation of a first shear wave generated inside the object by the first push signal; transmitting a detection ultrasound signal to the object in which the first shear wave is present and a second shear wave is generated by the second push signal; receiving a response signal in response to the detection ultrasound signal from the object; and generating an elastography image of the object based on the response signal.

According to another aspect of an exemplary embodiment, an ultrasound diagnosis apparatus includes a transmission unit configured to transmit a first push signal including one or more ultrasound signals to an object, to transmit a second push signal including one or more ultrasound signals to the object based on information regarding propagation of a first shear wave generated inside the object by the first push signal, and to transmit a detection ultrasound signal to the object in which the first shear wave is present and a second shear wave is generated by the second push signal; an ultrasound transmission/reception unit including a reception unit configured to receive a response signal in response to the detection ultrasound signal from the object; and an image processing unit configured to generate an elastography image of the object based on the response signal.

According to another aspect of an exemplary embodiment, a method of generating an elastography image includes transmitting a push signal including a plurality of ultrasound signals to an object, to stop sequentially transmitting of the plurality of ultrasound signals in a first direction, and to transmit a detection ultrasound signal to the object in which a shear wave is generated by transmitting the plurality of ultrasound signals; receiving a response signal in response to the detection ultrasound signal from the object; and generating an elastography image of the object based on the response signal.

According to another aspect of an exemplary embodiment, an ultrasound diagnosis apparatus includes a transmission unit configured to transmit a push signal including a plurality of ultrasound signals to an object, to stop sequentially transmitting of the plurality of ultrasound signals in a first direction, and to transmit a detection ultrasound signal to the object in which a shear wave is generated by transmitting the plurality of ultrasound signals; an ultrasound transmission/reception unit including a reception unit configured to receive a response signal in response to the detection ultrasound signal from the object; and an image processing unit configured to generate an elastography image of the object based on the response signal.

According to another aspect of an exemplary embodiment, a method of generating an elastography image includes transmitting repeatedly a push signal including one or more ultrasound signals to an object; transmitting a first detection ultrasound signal having a first time offset by transmitting the push signal to the object in which a shear wave is generated by the push signal; transmitting a second detection ultrasound signal having a second time offset by retransmitting the push signal to the object in which a shear wave is generated by retransmitting the push signal; acquiring first ultrasound image data from a first response signal in response to the first detection ultrasound signal and to acquire second ultrasound image data from a second response signal in response to the second detection ultrasound signal; and combining the first ultrasound image data and the second ultrasound image data based on the first time offset and the second time offset and to generate an elastography image of the object based on the combined ultrasound image data.

According to another aspect of an exemplary embodiment, an ultrasound diagnosis apparatus includes a transmission unit configured to transmit repeatedly a push signal including one or more ultrasound signals to an object, to transmit a first detection ultrasound signal having a first time offset by transmitting the push signal to the object in which a shear wave is generated by the push signal, and to transmit a second detection ultrasound signal having a second time offset by retransmitting the push signal to the object in which a shear wave is generated by retransmitting the push signal; an ultrasound transmission/reception unit including a reception unit configured to acquire first ultrasound image data from a first response signal in response to the first detection ultrasound signal and to acquire second ultrasound image data from a second response signal in response to the second detection ultrasound signal; and an image processing unit configured to combine the first ultrasound image data and the second ultrasound image data based on the first time offset and the second time offset and to generate an elastography image of the object based on the combined ultrasound image data.

According to another aspect of an exemplary embodiment, a method of generating an elastography image includes transmitting a push signal including one or more ultrasound signals to an object; transmitting a first detection ultrasound signal focused on a first scan line among a plurality of scan lines included in the object in which a shear wave is generated by the push signal; transmitting a second detection ultrasound signal focused on a second scan line among the plurality of scan lines; receiving a first response signal in response to the first detection ultrasound signal from the object and to receive a second response signal in response to the second detection ultrasound signal from the object; and generating an elastography image of the object by using the first response signal and the second response signal, to extract first image data corresponding to the first scan line from the first response signal, and to extract second image data corresponding to the second scan line from the second response signal, to combine the first image data and the second image data, and to generate a B mode image.

According to another aspect of an exemplary embodiment, an ultrasound diagnosis apparatus includes a transmission unit configured to transmit a push signal including one or more ultrasound signals to an object, to transmit a first detection ultrasound signal focused on a first scan line among a plurality of scan lines included in the object in which a shear wave is generated by the push signal, and to transmit a second detection ultrasound signal focused on a second scan line among the plurality of scan lines; an ultrasound transmission/reception unit including a reception unit configured to receive a first response signal in response to the first detection ultrasound signal from the object and to receive a second response signal in response to the second detection ultrasound signal from the object; and an image processing unit configured to generate an elastography image of the object by using the first response signal and the second response signal, to extract first image data corresponding to the first scan line from the first response signal, and to extract second image data corresponding to the second scan line from the second response signal, to combine the first image data and the second image data, and to generate a B mode image.

According to an aspect of another exemplary embodiment, a non-transitory computer-readable recording medium having recorded thereon a program for executing the method of generating an elastography image is provided.

BRIEF DESCRIPTION OF THE DRAWINGS

These and/or other aspects will become apparent and more readily appreciated from the following description of the exemplary embodiments, taken in conjunction with the accompanying drawings in which:

FIG. 1 is a diagram illustrating an ultrasound diagnosis apparatus according to an exemplary embodiment;
FIG. 2 is a diagram for describing a shear wave generated inside an object;
FIG. 3 is a block diagram of a configuration of an ultrasound diagnosis apparatus according to an exemplary embodiment;
FIGS. 4 and 5 are graphs of intensity maps and axial beam profiles of unfocused push signals;
FIG. 6 is a flowchart of a method of generating an elastography image according to an exemplary embodiment;
FIG. 7 is a diagram for describing unfocused push signals transmitted in different directions according to an exemplary embodiment;
FIG. 8 is a diagram for describing transmitted push signals according to an exemplary embodiment;
FIG. 9 is a diagram for describing an intensity map and an axial beam profile of unfocused push signals that are transmitted according to an exemplary embodiment;
FIG. 10 is a flowchart of a method of generating an elastography image according to an exemplary embodiment;
FIGS. 11 and 12 are diagrams for describing adaptively transmitted push signals according to an exemplary embodiment;
FIG. 13 is a flowchart of a method of generating an elastography image according to an exemplary embodiment;
FIG. 14 is a diagram for describing a method of applying a push signal so as to adjust a shape of a shear wave according to an exemplary embodiment;
FIGS. 15 and 16 are diagrams for describing a method of adjusting a shape of a shear wave generated by applying a push signal according to an exemplary embodiment;
FIG. 17 is a flowchart of a method of generating an elastography image according to an exemplary embodiment;
FIG. 18 is a diagram for describing a method of acquiring temporally decimated scan line image data so as to detect a shear wave according to an exemplary embodiment;

FIG. 19 is a diagram for describing a method of generating an elastography image by combining temporally decimated scan line image data according to an exemplary embodiment;

FIG. 20 is a diagram for describing a method of acquiring temporally decimated scan line image data so as to detect a shear wave according to an exemplary embodiment;

FIG. 21 is a diagram for describing a method of generating an elastography image by combining temporally decimated scan line image data according to an exemplary embodiment;

FIG. 22 is a diagram for describing a method of generating an elastography image over an entire image region by splitting an image region in a lateral direction and generating a plurality of partial elastography images according to an exemplary embodiment;

FIG. 23 is a diagram for describing a method of generating an elastography image at a quick processing speed by reducing a depth of an image region according to an exemplary embodiment;

FIG. 24 is a flowchart of a method of generating an elastography image according to an exemplary embodiment;

FIG. 25 is a diagram for describing a method of transmitting a detection ultrasound signal focused on a transmission reference scan line according to an exemplary embodiment;

FIG. 26 is a diagram for describing a method of generating an elastography image and a B mode image by shifting a transmission reference scan line of a detection ultrasound signal, acquiring image data, and combining the acquired image data according to an exemplary embodiment; and

FIG. 27 is a block diagram of a configuration of an ultrasound diagnosis apparatus according to an exemplary embodiment.

## DETAILED DESCRIPTION

Reference will now be made in detail to exemplary embodiments, examples of which are illustrated in the accompanying drawings, wherein like reference numerals refer to like elements throughout. In this regard, the present exemplary embodiments may have different forms and should not be construed as being limited to the descriptions set forth herein. Accordingly, the exemplary embodiments are merely described below, by referring to the figures, to explain aspects. Expressions such as "at least one of," when preceding a list of elements, modify the entire list of elements and do not modify the individual elements of the list.

Hereinafter, the terms used in the specification will be briefly described, and then exemplary embodiments will be described in detail.

The terms used in this specification are those general terms currently widely used in the art, but the terms may vary according to the intention of those of ordinary skill in the art, precedents, or new technology in the art. Also, specified terms may be selected by the applicant, and in this case, the detailed meaning thereof will be described in the detailed description. Thus, the terms used in the specification should be understood not as simple names but based on the meaning of the terms and the overall description.

Throughout the specification, it will also be understood that when a component "includes" an element, unless there is another opposite description thereto, it should be understood that the component does not exclude another element but may further include another element. In addition, terms such as "... unit", "... module", or the like refer to units that perform at least one function or operation, and the units may be implemented as hardware or software or as a combination of hardware and software.

Throughout the specification, an "ultrasonic image" refers to an image of an object obtained using an ultrasonic wave. Furthermore, in the present specification, "object" may include a person or an animal, or a part of a person or an animal. For example, the object may include the liver, the heart, the womb, the brain, a breast, the abdomen, or a blood vessel.

Furthermore, the "object" may include a phantom. The phantom means a material having a volume that is approximately the intensity and effective atomic number of a living thing, and may include a spherical phantom having a property similar to a human body.

Furthermore, in the present specification, "user" refers to a medical professional, such as a doctor, a nurse, a medical laboratory technologist, and an engineer who repairs a medical apparatus, but the user is not limited thereto.

Reference will now be made in detail to embodiments, examples of which are illustrated in the accompanying drawings. In this regard, the present exemplary embodiments may have different forms and should not be construed as being limited to the descriptions set forth herein. In the following description, well-known functions or constructions are not described in detail since they would obscure the exemplary embodiments with unnecessary detail.

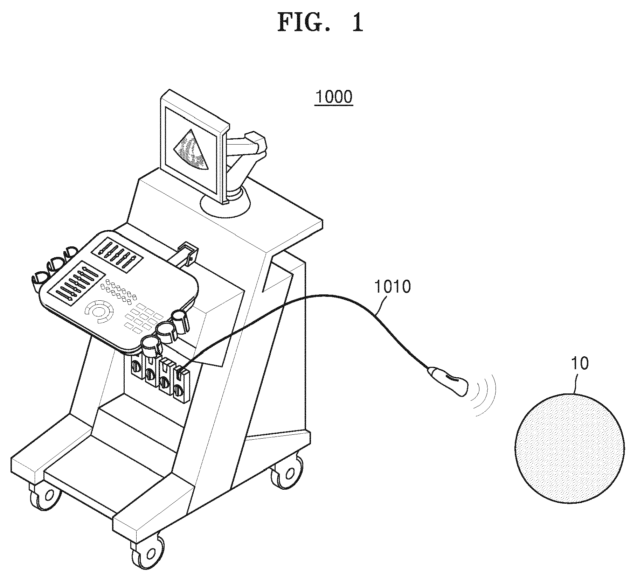

FIG. 1 is a diagram illustrating an ultrasound diagnosis apparatus 1000 according to an exemplary embodiment.

As illustrated in FIG. 1, the ultrasound diagnosis apparatus 1000 may transmit an ultrasound signal to an object 10 through a probe 1010. The ultrasound diagnosis apparatus 1000 may receive an ultrasound echo signal reflected from the object 10 to generate an ultrasound image.

In the present specification, an ultrasound image may be implemented in various ways. For example, the ultrasonic image may include at least one of a brightness (B) mode image in which a level of an ultrasonic echo signal reflected from an object is expressed as brightness, a color Doppler image in which a speed of a moving object is expressed as a color by using the Doppler effect, a spectral Doppler image in which an image of a moving object is expressed as a spectrum type by using the Doppler effect, and a motion (M) mode image that shows a motion of an object over time at a certain place, but is not limited thereto. According to an exemplary embodiment, the ultrasound image may be a two-dimensional (2D) image, a three-dimensional (3D) image, or a four-dimensional (4D) image.

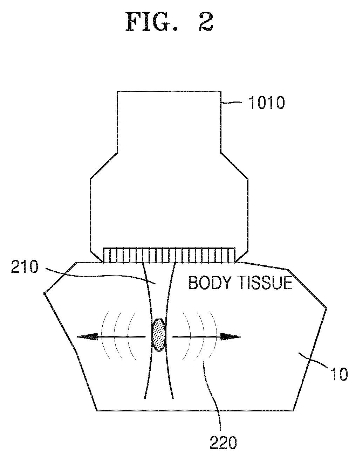

According to an exemplary embodiment, the ultrasound diagnosis apparatus 1000 may provide an elastography image that shows an elasticity contrast of the object by using an acoustic radiation force. The elastography image provided by the ultrasound diagnosis apparatus 1000 according to an exemplary embodiment will be described in detail with reference to FIG. 2.

FIG. 2 is a diagram for describing a shear wave 220 generated inside the object 10.

As illustrated in FIG. 2, the ultrasound diagnosis apparatus 1000 may transmit a signal 210 (for convenience of description, hereinafter referred to as a "push signal") for pushing a partial region of the object 10, to the object 10.

To push the partial region to the object 10 means to apply pressure to the partial region by generating an acoustic radiation force inside the object 10. For example, the ultrasound diagnosis apparatus 1000 may transmit the push signal 210 to the object 10 by using transducer elements of the probe 1010. The push signal 210 may be configured as a plurality of ultrasound signals transmitted into the object 10. The push signal 210 may be configured in the form of a beam including ultrasound beams having directionality.

The ultrasound diagnosis apparatus 1000 may apply the acoustic radiation force into the object 10 and acquire a propagation characteristic of the shear wave 220 generated by the applied acoustic radiation force, thereby digitizing and imaging elasticity of a tissue.

According to an exemplary embodiment, the ultrasound diagnosis apparatus 1000 may generate a spherical shear wave by transmitting the push signal 210 focused on a point inside the object 10, may generate an oblique shear wave by sequentially transmitting the push signals 210 focused on a plurality of points inside the object 10, or may generate a plane shear wave traveling in a direction perpendicular to a direction in which the push signal 210 is transmitted by transmitting the unfocused push signal 210, by using the transducer elements of the probe 1010. The unfocused push signal 210, which is a signal that is not focused on one specific point, may be a signal transmitted from at least a part of the transducer elements included in the probe 1010. The unfocused push signal 210 may be a signal including ultrasound signals focused on infinite number of points inside the object 10. For example, the unfocused push signal 210 may be a signal focused on a particular line inside the object 10 or configured as ultrasound signals focused on a particular plane inside the object 10.

If the ultrasound diagnosis apparatus 1000 transmits the push signal 210, the shear wave 220 may be generated with respect to a region pushed by the push signal 210 inside the object 10.

When the push signal 210 focused on the point inside the object 10 is transmitted, since the spherical shear wave is formed and propagates in a relatively small region in an axial direction and a lateral direction of the push signal 210, a size of a region of interest (ROI) may be limited. When high voltages are applied to transducer elements that generate the push signal 210 or a greater number of transducer elements are used in order to increase the size of the ROI, a problem of stability may occur.

When the push signals 210 focused on a plurality of points located in the axial direction are sequentially transmitted, wave planes of spherical shear waves generated in the plurality of points may be added to form the plane shear wave traveling in an oblique direction. In this case, it is possible to set a wide ROI compared to a case where the push signal 210 focused on the point is transmitted. However, the shear wave may not be observed at a location where the push signal 210 is focused and transmitted, and if the push signal 210 is away from the location where the push signal 210 is transmitted, the shear wave may be attenuated. Thus, if the push signal 210 is away from the location where the push signal 210 is transmitted, since the shear wave may be attenuated, an error may occur or estimation itself may be impossible when an elastography image is generated based on the shear wave.

Meanwhile, the ultrasound diagnosis apparatus 1000 according to an exemplary embodiment may operate for partial transducer elements to transmit the unfocused push signal 210 that is not focused on a specific point. Partial transducer elements that transmit the unfocused push signal 210 may consecutively transmit the push signals 210 at different depths in the axial direction in which the push signal 210 is transmitted, to regions inside the object 10. For example, a case where the ultrasound diagnosis apparatus 1000 according to an exemplary embodiment transmits the unfocused push signal 210 is illustrated in FIG. 2. In this case, the shear wave may propagate in the direction perpendicular to the direction in which the push signal 210 is transmitted. Thus, the ultrasound diagnosis apparatus 1000 may observe the shear wave a left region to a right region of a point where the push signal 210 is arrived as deep as possible as the ROI by transmitting the push signal 210 once.

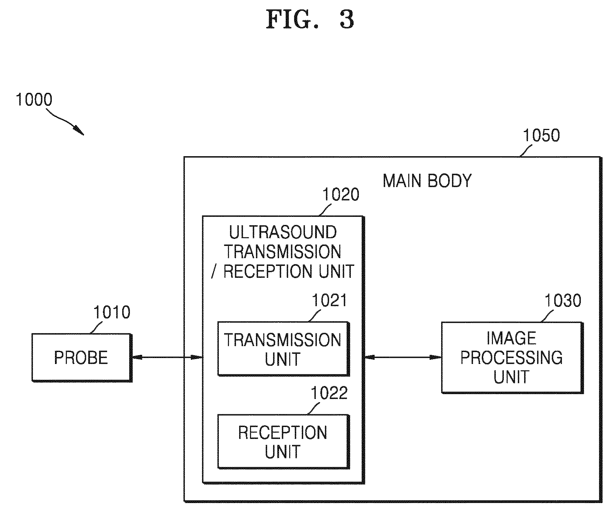

FIG. 3 is a block diagram of a configuration of the ultrasound diagnosis apparatus 1000 according to an exemplary embodiment.

As illustrated in FIG. 3, the ultrasound diagnosis apparatus 1000 according to an exemplary embodiment may include the probe 1010 and a main body 1050. The main body 1050 may include an ultrasound transmission/reception unit 1020 and an image processing unit 1030. The ultrasound transmission/reception unit 1020 according to an exemplary embodiment may include a transmission unit 1021 that transmits a detection ultrasound signal and a reception unit 1022 that receives a response signal to the detection ultrasound signal to transmit a push signal and acquire a propagation characteristic of a shear wave generated by the push signal in order to generate the shear wave.

The probe 1010 may transmit ultrasound waves to an object based on a driving signal applied by the ultrasound transmission/reception unit 1020 and receive echo signals reflected by the object. The probe 1010 may include a plurality of transducers, and the plurality of transducers oscillate based on electric signals transmitted thereto and generate acoustic energy, that is, ultrasound waves. The probe 1010 may be connected to the main body of the ultrasound apparatus 1000 by wired or wirelessly. According to an exemplary embodiment, the ultrasound diagnosis apparatus 1000 may include a plurality of the probes 1010. According to an exemplary embodiment, the probe 1010 may include at least one of a 1D probe, a 1.5D probe, a 2D (matrix) probe, and a 3D probe.

According to an exemplary embodiment, the probe 1010 may transmit a push signal to the object, thereby inducing a shear wave. The probe 1010 may transmit the detection ultrasound signal for tracking the shear wave to the object, and receive the response signal to the detection ultrasound signal from the object.

The ultrasound transmission/reception unit 1020 may control the probe 1010 to transmit the push signal to the object, transmit the detection ultrasound signal, and receive the response signal from the object. The ultrasound transmission/reception unit 1020 may include the transmission unit 1021 and the reception unit 1022.

The transmission unit 1021 may control the plurality of transducer elements included in the probe 1010 to transmit the push signal to the object.

The transmission unit 1021 may generate a spherical shear wave by transmitting a push signal focused on a point inside the object, may generate an oblique shear wave by sequentially transmitting push signals focused on a plurality of points inside the object, or may generate a plane shear wave traveling in a direction perpendicular to a direction in which the push signal is transmitted by transmitting the unfocused push signal, by using the transducer elements of the probe 1010.

The transmission unit 1021 according to an exemplary embodiment may transmit the push signal to the object in various ways to generate a strong shear wave with respect to a wide region inside the object.

As an example, the transmission unit 1021 according to an exemplary embodiment may generate the shear wave by transmitting unfocused push signals steered at different angles from the plurality of transducer elements to the object.

The transmission unit 1021 may adjust an electrical signal applied to each of the transducer elements of the probe 1010, thereby changing a phase of an ultrasound signal transmitted by each of the transducer elements or allowing the ultrasound signal to have a time delay. Steering means that the transmission unit 1021 changes a direction in which the ultrasound signal is transmitted due to a phase change or the time delay with respect to the ultrasound signal transmitted by each of the transducer elements.

That is, when the unfocused push signals include a plurality of ultrasound signals, the transmission unit 1021 may change a direction in which the push signal is transmitted by adjusting a phase or a time delay of each of the ultrasound signals. That is, the transmission unit 1021 may steer the push signal at a particular angle.

As another example, the transmission unit 1021 according to an exemplary embodiment may generate a first shear wave inside the object by transmitting a first push signal including at least one ultrasound signal to the object. The transmission unit 1021 may transmit a second push signal including at least one ultrasound signal to the object based on information regarding the propagation of the first shear wave. The transmission unit 1021 may generate the second shear wave to interfere constructively with the first shear wave.

As another example, the transmission unit 1021 according to an exemplary embodiment may transmit the push signal including the plurality of push signals to the object. For example, the push signal may be in the form of a push beam including a plurality of ultrasound beams arranged in an axial direction. The ultrasound beams may have beam widths smaller than the push beam.

The transmission unit 1021 may generate the shear wave by sequentially stopping transmitting the ultrasound beams in a lateral direction. That is, the plurality of ultrasound beams may start being simultaneously transmitted by the transmission unit 1021 and may be transmitted during different cycles of time. Time cycles of the plurality of ultrasound beams may increase or decrease in the lateral direction in which the plurality of ultrasound beams are arranged. For example, the transmission unit 1021 may stop transmitting the ultrasound beams sequentially from an ultrasound beam push signal located at an edge of the ultrasound beams in the lateral direction.

In addition, the transmission unit 1021 according to an exemplary embodiment may transmit the detection ultrasound signal for tracking the shear wave to the object in which the shear wave is generated by the push signal.

For example, the transmission unit 1021 may transmit the ultrasound signal to the object in which the shear wave is present and receive a response signal of the transmitted ultrasound signal from the object so as to track the shear wave generated by the push signal. The transmission unit 1021 may control the plurality of transducer elements included in the probe 1010 to transmit and receive the detection ultrasound signal so as to track the shear wave.

The transmission unit 1021 may transmit the detection ultrasound signal to the object by using the transducer elements of the probe 1010.

Meanwhile, the reception unit 1022 may receive, from the object, the response signal of the detection ultrasound signal transmitted to the object. The reception unit 1022 may control the plurality of transducer elements included in the probe 1010 to transmit and receive the ultrasound signal so as to track the shear wave. Ultrasound image data may be acquired from the received response signal.

The ultrasound transmission/reception unit 1020 according to an exemplary embodiment may transmit and receive the ultrasound signal at a relatively low frame rate, thereby transmitting and receiving the ultrasound signal to the object in various ways so as to acquire the propagation characteristic of the shear wave.

As an example, the ultrasound transmission/reception unit 1020 according to an exemplary embodiment may transmit a first detection ultrasound signal having a first time offset to the object in which the shear wave is present and acquire first ultrasound image data from a first response signal to the first detection ultrasound signal. The reception unit 1022 may transmit a second detection ultrasound signal having a second time offset to the object in which the shear wave is present and acquire second ultrasound image data from a second response signal to the second detection ultrasound signal.

As another example, the ultrasound transmission/reception unit 1020 according to an exemplary embodiment may transmit a first ultrasound signal focused on a first scan line among a plurality of scan lines included in the object and receive a first response signal to the first ultrasound signal. The reception unit 1022 may transmit a second ultrasound signal focused on a second scan line among the plurality of scan lines and receive a second response signal to the second ultrasound signal.

The image processing unit 1030 may generate an elastography image of the object based on the response signal received from the reception unit 1022.

As an example, the image processing unit 1030 may acquire a plurality of ultrasound image frames with respect to the object based on the response signal received from the reception unit 1022. The image processing unit 1030 may calculate a displacement of the object presented by the ultrasound image frames by comparing the plurality of ultrasound image frames. The image processing unit 1030 may acquire information regarding the elasticity of a tissue included in the object based on the calculated displacement between the ultrasound image frames-> ok.

For example, the information regarding the elasticity may include strain, elasticity, etc. The strain means a ratio of a length variation of the object after tissue is deformed by stress to a length of the tissue before the tissue is deformed. The elasticity means a ratio of stress to strain.

The image processing unit 1030 may generate the elastography image that indicates information regarding the elasticity of tissue included in the object using at least one of a color, brightness, and a diagram.

As another example, the image processing unit 1030 may measure a shear wave parameter indicating a characteristic of the shear wave based on the response signal received from the object. The shear wave parameter may include at least one of a propagation velocity Vs of the shear wave and an attenuation coefficient $\alpha$ thereof.

The propagation velocity Vs of the shear wave may be acquired according to Equation 1 below. The attenuation coefficient $\alpha$ of the shear wave may be acquired according to Equation 2 below. In Equations 1 and 2, R and X, respectively, denote real and imaginary components of acoustic impedance, $\rho$ denotes a density of the object, and $\omega$ denotes an angular frequency of the shear wave.

[Equation 1]

$$V_S = \frac{R^2 + X^2}{\rho R}$$

[Equation 2]

$$\alpha = \frac{\rho \omega X}{(R^2 + X^2)}$$

Meanwhile, the ultrasound transmission/reception unit 1020 may transmit, several times, an ultrasound signal to a region in which the shear wave propagates and may receive, several times, a response signal to the ultrasound signal transmitted several times from the object. The image processing unit 1030 may measure the shear wave parameter by applying cross-correlation to the response signal received several times. In addition to the above-described methods, the shear wave characteristic induced inside the object may be measured in various ways obvious to one or ordinary skill in the art.

The image processing unit 1030 may acquire an elasticity characteristic of the object using the shear wave parameter.

The elasticity characteristic of the object may include at least one of a shear modulus G of the object, a young's modulus E thereof, and a shear viscosity $\eta$ thereof.

The shear modulus G of the object may be acquired according to Equation 3 below. The young's modulus E of the object may be acquired according to Equation 4 below. The shear viscosity $\eta$ of the object may be acquired according to Equation 5 below.

[Equation 3]

$$G = \frac{(R^2 - X^2)}{\rho}$$

[Equation 4]

$$E = 3G$$

[Equation 5]

$$\eta = \frac{2RX}{\omega\rho}$$

The image processing unit 1030 according to an exemplary embodiment may generate the elastography image that indicates the elasticity characteristic of the object using at least one of a color, brightness, and a diagram. For example, the image processing unit 1030 may map the elasticity characteristic of the object to a black and white scale or a color scale to generate the elastography image of the object.

The image processing unit 1030 according to an exemplary embodiment may generate not only the elasticity characteristic of the object but also a B mode image indicating the object. The image processing unit 1030 may calculate a reflection coefficient from the response signal to the ultrasound signal transmitted to the object and generate the mode image indicating the calculate reflection coefficient. The B mode image may indicate the reflection coefficient calculated from the response signal using brightness of each pixel included in the B mode image.

For example, the image processing unit 1030 may generate the elastography image using the first response signal to the first ultrasound signal that is transmitted after being focused on the first scan line among the plurality of scan lines included in the object and receive a first response signal to the first ultrasound signal and the second response signal to the second ultrasound signal that is transmitted after being focused on the second scan line among the plurality of scan lines. The image processing unit 1030 may extract first image data corresponding to the first scan line from the first response signal, extract second image data corresponding to the second scan line from the second response signal, and combine the first image data and the second image data to generate the B mode image.

Meanwhile, the ultrasound diagnosis apparatus 1000 according to an exemplary embodiment may operate to allow partial transducer elements to transmit the unfocused push signal that is not focused on a specific point. Partial transducer elements that transmit the unfocused push signal may consecutively transmit the push signal at different depths in the axial direction. In this case, the shear wave may propagate in a direction perpendicular to the direction in which the push signal is transmitted. Thus, the ultrasound diagnosis apparatus 1000 may observe the shear wave from a left region to a right region of a point where the push signal is arrived as deep as possible as the ROI by transmitting the push signal once.

The axial direction, which is a direction perpendicular to a direction in which transducer elements are arranged, may be a direction in which an ultrasound signal that is not steered is transmitted from a transducer element. For example, the axial direction may be a depth direction of the object.

The acoustic radiation force used to generate the shear wave is in proportion to the intensity of a push signal. In the case of an unfocused push signal, an intensity profile is not uniform in an axial direction of the unfocused push signal, which causes a phenomenon that the intensity of the unfocused push signal varies depending on a depth. In other words, a size and a shape of the shear wave generated by the unfocused push signal vary depending on the depth, which may cause an error in a velocity estimate obtained on the basis of an assumption that the shear wave uniformly travels left and right.

Upon comparing the intensity of a focused push signal in which energy of a push beam is focused on a point with the intensity of the unfocused push signal, since the intensity of the unfocused push signal is low, the intensity of the shear wave generated by the unfocused push signal is also low. Thus, when the shear wave is generated using the unfocused push signal, since the intensity of the shear wave is low, a signal-noise ratio (SNR) is disadvantageously reduced when tracking the shear wave. To increase the SNR, if the number of transducer elements used to transmit the push signal increases, since a beam width of the push signal increases, a phenomenon that an axial beam profile of the push signal is not uniform further deteriorates.

Therefore, to solve the above-described problem, the ultrasound diagnosis apparatus 1000 according to an exemplary embodiment may transmit a push signal in which an axial beam profile is uniform (i.e., the beam profile is uniform at different depths). The ultrasound diagnosis apparatus 1000 according to an exemplary embodiment may maintain an appropriately narrow beam of the push signal width although using a great number of transducer elements, thereby preventing the energy of push signal from being diffused. The ultrasound diagnosis apparatus 1000 according to an exemplary embodiment may amplify the intensity of the shear wave, thereby increasing the SNR. The ultrasound diagnosis apparatus 1000 according to an exemplary embodiment may actively control a shape and property of the shear wave.

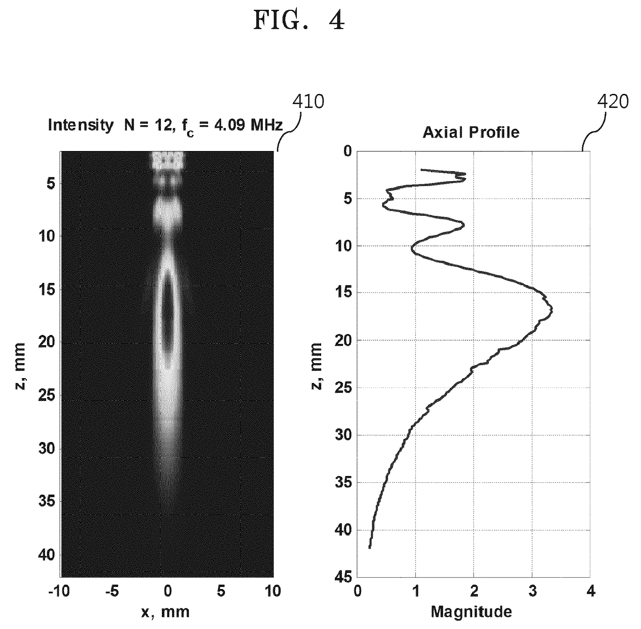

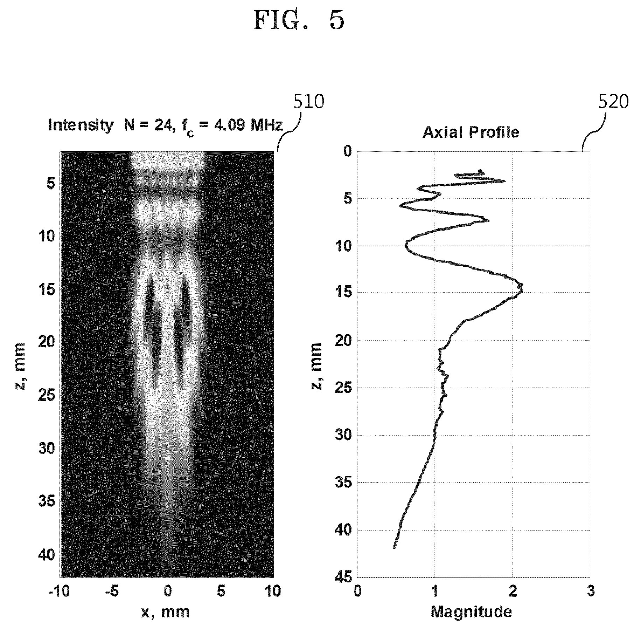

FIGS. 4 and 5 are graphs of intensity maps 410 and 510 and axial beam profiles 420 and 520 of unfocused push signals.

The intensity maps 410 and 510 of FIGS. 4 and 5 are maps illustrating the intensity of the unfocused push signals as colors. The axial beam profiles 420 and 520 of FIGS. 4 and 5 are maps illustrating the intensity of the unfocused push signals applied to an object along a central axis of the unfocused push signals. The central axis of the unfocused push signals is an axis on a transducer element located in a center of an array in which a plurality of transducer elements that transmit the unfocused push signals are arranged, and means an axis parallel to an axial direction. In the axial beam profiles 420 and 520, an x axis of denotes a relative intensity of an ultrasound signal, and a y axis denotes a depth.

FIG. 4 illustrates the intensity map 410 and the axial beam profile 420 of the unfocused push signals transmitted using transducer elements. FIG. 5 illustrates the intensity map 510 and the axial beam profile 520 of the unfocused push signals transmitted using 24 transducer elements.

As shown in FIGS. 4 and 5, when the ultrasound diagnosis apparatus 1000 uses the unfocused push signals to generate a shear wave, a greater number of transducer elements does not result in a higher intensity shear wave. Instead, the greater number of transducer elements results in the unfocused push signals having a diffused shape, which causes an error in estimating a propagation velocity of the shear wave.

The ultrasound diagnosis apparatus 1000 according to an exemplary embodiment may transmit a push signal in which an axial beam profile is uniform (i.e., the beam profile is uniform at different depths). The ultrasound diagnosis apparatus 1000 according to an exemplary embodiment may maintain an appropriately narrow beam width of the push signal although using a great number of transducer elements, thereby preventing the energy of the push signal from being diffused.

As an example, the ultrasound diagnosis apparatus 1000 according to an exemplary embodiment may set a plurality of focal points at an axis of the axial direction and focus subsets of transducer elements on the focal points of different depths to transmit the push signals. For example, the ultrasound diagnosis apparatus 1000 may form two steered and unfocused push signals facing a central axis (an axis of the axial direction on a center of an array in which transducer elements are arranged) such that the energy of the push signals may be collected at the plurality of focal points on the central axis.

As another example, the ultrasound diagnosis apparatus 1000 according to an exemplary embodiment may apply an apodization function-> ok to transducer elements to transmit push signals having different intensities according to a depth.

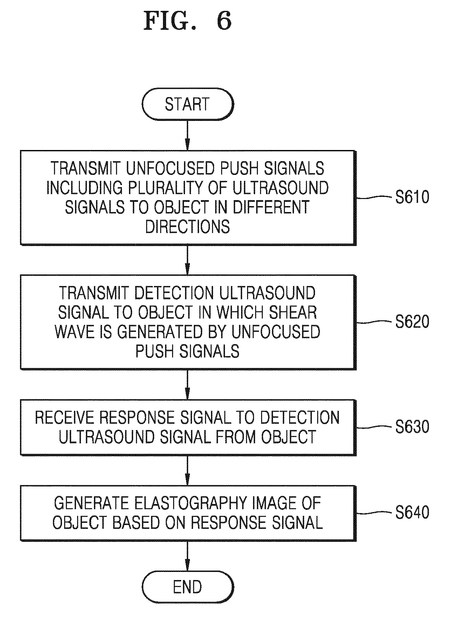

A detailed method for the ultrasound diagnosis apparatus 1000 according to an exemplary embodiment to maintain the appropriately narrow beam width of the push signal and prevent the energy of push signal from being diffused will now be described with reference to FIGS. 6 through 9 below.

FIG. 6 is a flowchart of a method of generating an elastography image according to an exemplary embodiment.

The method of generating the elastography image of FIG. 6 may be performed by the ultrasound diagnosis apparatus 1000 of FIG. 2. In more detail, operation S610 may be performed by the probe 1010 and the transmission unit 1021 of FIG. 2, operations S620 and S630 may be performed by the probe 1010 and the shear wave information detection unit 1022 of FIG. 2, and operation S640 may be performed by the image processing unit 1030 of FIG. 2. Thus, the description of FIG. 2 may be applied to the method of generating the elastography image of FIG. 6, and redundant descriptions are omitted.

In operation S610, the ultrasound diagnosis apparatus 1000 according to an exemplary embodiment may transmit unfocused push signals including a plurality of ultrasound signals to an object in different directions.

The ultrasound diagnosis apparatus 1000 may steer the unfocused push signals at different angles, thereby transmitting the unfocused push signals to the object in different directions.

An unfocused push signal may include a plurality of ultrasound signals transmitted to a plurality of focal points inside the object. The unfocused push signal may generate a shear wave inside the object by an acoustic radiation force generated at the plurality of focal points inside the object. The unfocused push signal may mean a plane wave signal that pushes the object by generating the acoustic radiation force. For example, the ultrasound diagnosis apparatus 1000 may split a plurality of transducer elements into a plurality of subnets, focus the subnets at focal points of different depths, and transmit ultrasound signals to transmit the unfocused push signal.

The ultrasound diagnosis apparatus 1000 may apply an electrical signal to each of the plurality of transducer signals to allow each transducer element to output an ultrasound signal to the object based on the electrical signal applied to each transducer element. The ultrasound diagnosis apparatus 1000 may adjust the electrical signal to each transducer signal to change a phase of the ultrasound signal transmitted by each transducer element or allow the ultrasound signal to have a time delay. Steering refers to the ultrasound diagnosis apparatus 1000 changing a direction of a push signal due to a phase change or the time delay with respect to ultrasound signals transmitted by the plurality of transducer elements.

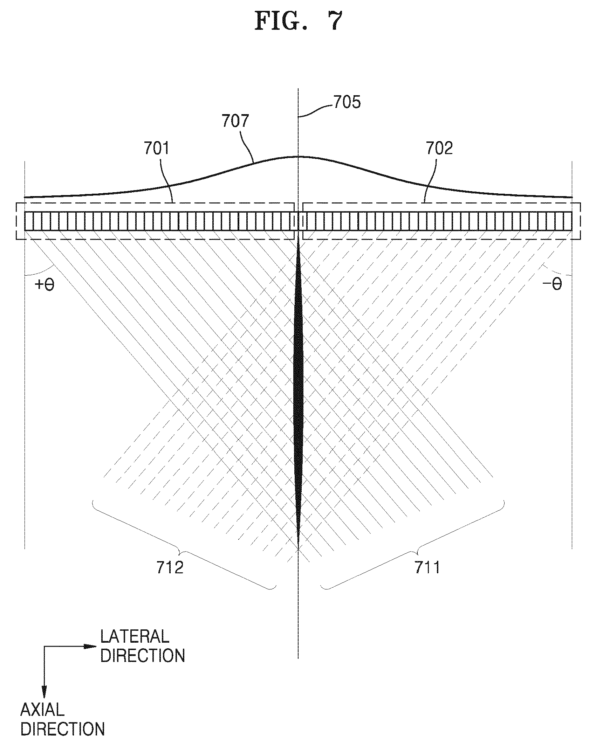

The ultrasound diagnosis apparatus 1000 may transmit the unfocused push signals to the object in different directions such that the unfocused push signals may simultaneously arrive on a predetermined axis. The transducer elements have different distances with respect to the predetermined axis, and thus time taken for the ultrasound signals transmitted by the plurality of transducer elements to arrive on the predetermined axis may be different. Therefore, the ultrasound diagnosis apparatus 1000 may change the phase or the time delay of the ultrasound signal transmitted by each transducer element, thereby transmitting two unfocused push signals 711 and 712 in different directions as shown in FIG. 7. That is, the ultrasound diagnosis apparatus 1000 may steer and transmit two unfocused push signals.

The ultrasound diagnosis apparatus 1000 may steer and transmit push signals such that constructive interference may occur between two push signals on a predetermined axis parallel to an axial direction of a plurality of transducer elements.

The ultrasound diagnosis apparatus 1000 may transmit a first unfocused push signal transmitted from a first group of adjacent transducer elements among a plurality of transducer elements included in the probe 1010. The ultrasound diagnosis apparatus 1000 may transmit a second unfocused push signal transmitted from a second group of adjacent transducer elements among the plurality of transducer elements included in the probe 1010.

According to an exemplary embodiment, the first unfocused push signal and the secondly unfocused push signal may be transmitted in different directions such that the firstly and second unfocused push signals may be symmetrical to each other with respect to a predetermined axis. The predetermined axis may be a center axis on a center of an array in which a plurality of transducer elements are arranged and parallel to an axial direction of the plurality of transducer elements. For example, the first unfocused push signal may be transmitted in a direction inclined by $+\theta°$ with respect to the predetermined axis, and the second unfocused push signal may be transmitted in a direction inclined by $-\theta°$ with respect to the predetermined axis.

The ultrasound diagnosis apparatus 1000 according to an exemplary embodiment may transmit at least two unfocused push signals having intensity to which an apodization function is applied to the object with respect to the center of the array in which the plurality of transducer elements are arranged. That is, the unfocused push signals transmitted from the ultrasound diagnosis apparatus 1000 may include a plurality of ultrasound signals transmitted from the respective plurality of transducer elements. The plurality of transducer elements may transmit the plurality of ultrasound signals of which intensity is reduced from a transducer element located at the center of the array in which the plurality of transducer elements are arranged to a transducer element located at an end thereof.

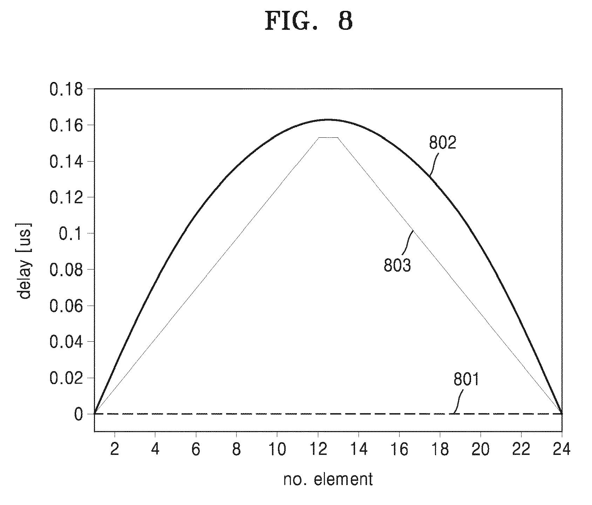

According to an exemplary embodiment, a method of transmitting the at least two unfocused push signals will be described in detail with reference to FIGS. 7 and 8 later.

Operations S620 and S630, the ultrasound diagnosis apparatus 1000 may transmit a detection ultrasound signal to the object in which a shear wave is generated by the unfocused push signals transmitted in operation S610 and receive a response signal to the detection ultrasound signal from the object.

In operation S640, the ultrasound diagnosis apparatus 1000 may generate the elastography image of the object based on the response signal received in operation S620.

For example, the ultrasound diagnosis apparatus 1000 may determine a propagation velocity of the shear wave in each region inside the object using the response signal. The ultrasound diagnosis apparatus 1000 may generate the elastography image using the propagation velocity of the shear wave.

A general elasticity of a body tissue is in proportion to a square of the propagation velocity of the shear wave, and thus the propagation velocity of the shear wave is faster in a tumor having a higher elasticity than that of normal tissue. The ultrasound diagnosis apparatus 1000 may generate the elastography image by mapping the propagation velocity of the shear wave or a shear modulus thereof to a color.

According to an exemplary embodiment, the ultrasound diagnosis apparatus 1000 may display the elastography image overlapped on a B mode image. In this regard, according to an exemplary embodiment, the ultrasound diagnosis apparatus 1000 may display the elastography image on the B mode image in a semi-transparent state.

FIG. 7 is a diagram for describing unfocused push signals transmitted in different directions according to an exemplary embodiment.

As shown in FIG. 7, according to an exemplary embodiment, a plurality of transducer elements 701 and 702 may be divided into the first group 701 and the second group 702 that respectively include a same number of adjacent transducer elements with respect to a center of an array in which the plurality of transducer elements 701 and 702 are arranged.

In this regard, the first unfocused push signal 711 transmitted from the first group 701 and the secondly unfocused push signal 712 transmitted from the second group 702 may be transmitted in different directions such that the firstly and second unfocused push signals 711 and 712 may be symmetrical to each other with respect to a predetermined axis 705. For example, the first and second unfocused push signals 711 and 712 may be steered in a size of the same angle-> ok in an opposite direction with respect to an axial direction of the plurality of transducer elements 701 and 702.

FIG. 7 illustrates the two unfocused push signals 711 and 712 that are respectively steered at $+\theta°$ and $-\theta°$.

As shown in FIG. 7, the ultrasound diagnosis apparatus 1000 according to an exemplary embodiment may transmit the unfocused push signals 711 and 712 that are steered to arrive on the predetermined axis 705 for the same period of time, thereby narrowing lateral widths of the unfocused push signals 711 and 712.

Meanwhile, the ultrasound diagnosis apparatus 1000 according to an exemplary embodiment may adjust and transmit a push signal such that an axial profile of the push signal may be uniform. As shown in the axial beam profiles 420 and 520 of FIGS. 4 and 5, a size of a shear wave generated by the push signal may vary according to a depth inside an object due to a non-uniformly formed intensity profile of the push signal.

Therefore, since a propagation velocity of the shear wave traveling in a direction (i.e. a lateral direction) perpendicular to a direction in which the push signal is applied varies according to the depth, an estimate of the propagation velocity of the shear wave calculated on the assumption of a time-invariant plane wave front has an error. Meanwhile, if the axial beam profile of the push signal is uniform, since a plane shear wave traveling at a uniform velocity in a lateral direction within a depth of interest (DOI) may be assumed, a more accurate propagation velocity may be calculated according to the depth.

Therefore, the ultrasound diagnosis apparatus 1000 according to an exemplary embodiment may adjust at least one of amplitude and a time delay of an electrical signal applied to each of transducer elements used to transmit the push signal, and thus the axial bema profile of the push signal may be uniform. Alternatively, the ultrasound diagnosis apparatus 1000 according to an exemplary embodiment may apply different electrical signals to the transducer elements, and thus the axial bema profile of the push signal may be uniform.

For example, the ultrasound diagnosis apparatus 1000 according to an exemplary embodiment may transmit at least two unfocused push signals to which an apodization function is applied to the object with respect to a center of an array in which a plurality of transducer elements are arranged. The ultrasound diagnosis apparatus 1000 may adjust the intensity of the push signal according to the depth by applying the apodization function.

As shown in the axial beam profiles 420 and 520 of FIGS. 4 and 5, the energy of the push signal may be focused at a specific depth, which may show that the intensity of the push signal may be relatively weak at a near depth from a surface of the object.

Therefore, to solve this, the ultrasound diagnosis apparatus 1000 according to an exemplary embodiment may apply the apodization function to a transducer element located at the center of the array in which the plurality of transducer elements that transmit the push signal are arranged. For example, a function in which a value is tapered from the center to both ends like a Hanning window function may be used as the apodization function. For example, the apodization function having a length equal, 1.25 times, 1.5 times, or 2 times a length of the transducer elements used to transmit the push signal may be used.

As shown in a graph 707 of FIG. 7, the ultrasound diagnosis apparatus 1000 according to an exemplary embodiment may apply the apodization function to the intensity of ultrasound signals transmitted from the plurality of transducer elements. The ultrasound diagnosis apparatus 1000 may transmit the ultrasound signals of which intensity is reduced from the transducer element located at the center of the array in which the plurality of transducer elements are arranged to a transducer element located at an end thereof. According to an exemplary embodiment, electrical signals of which intensity--> ok is relatively reduced toward both ends with respect to the transducer element located at the center may be applied to the transducer elements, and thus the axial bema profile of the push signal transmitted by the transducer elements may be uniform.

As shown in FIG. 8, the ultrasound diagnosis apparatus 1000 according to an exemplary embodiment may apply a time delay to a plurality of transducer elements, thereby transmitting a steered push signal.

A graph 801 illustrates the time delay applied to each transducer element when the ultrasound diagnosis apparatus 1000 transmits an unfocused push signal that is not steered from the plurality of transducer elements to an object. As shown in FIG. 8, to transmit the unfocused push signal that is not steered, the ultrasound diagnosis apparatus 1000 may not apply the time delay to all the transducer elements.

A graph 802 illustrates the time delay applied to each transducer element when the ultrasound diagnosis apparatus 1000 transmits an unfocused push signal that is focused on a point inside an object from the plurality of transducer elements to the object.

A graph 803 illustrates the time delay applied to each transducer element when the ultrasound diagnosis apparatus 1000 according to an exemplary embodiment transmits two unfocused push signals in different directions such that constructive interference between the two unfocused push signals may occur on a center axis. As shown in the graph 803, the ultrasound diagnosis apparatus 1000 may apply the time delay to the plurality of transducer elements such that the time delay applied to each transducer element may be symmetrical to each other with respect to a center device and the time delay applied to adjacent transducer elements may have a uniform difference. When the time delay is applied to the plurality of transducer elements as shown in the graph 803, the two unfocused push signals transmitted from the ultrasound diagnosis apparatus 1000 may be steered at different angles such that the two unfocused push signals may be symmetrical to each other on a center axis.

FIG. 8 illustrates the time delay applied to each transducer element when 24 transducer elements are used to transmit push signals but the exemplary embodiments are not limited thereto. Various numbers of transducer elements may be used to transmit push signals.

As shown in FIGS. 4 and 5, if a small number of transducer elements is used, a lateral width of an unfocused push signal is reduced. However, if a small number of transducer elements is used, since the intensity of the unfocused push signal is reduced, there is a problem that an SNR deteriorates.

Meanwhile, according to an exemplary embodiment, a push signal having a narrow width and a high intensity may be transmitted. According to an exemplary embodiment, irrespective of the number of used transducer elements, since the push signal having the narrow width may be transmitted, the number of available transducer elements is not limited. Thus, according to an exemplary embodiment, a greater number of transducer elements may be used to generate a higher intensity shear wave.

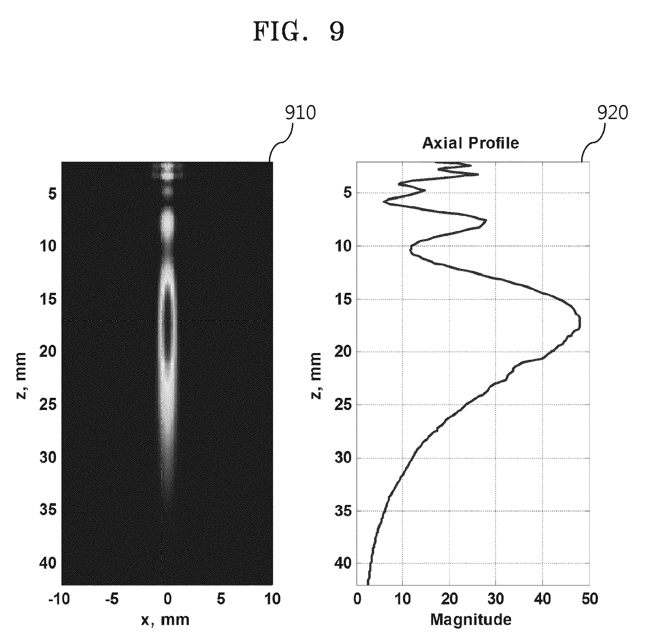

FIG. 9 is a diagram for describing an intensity map 910 and an axial beam profile 920 of unfocused push signals that are transmitted according to an exemplary embodiment. As shown in FIG. 9, the ultrasound diagnosis apparatus 1000 according to an exemplary embodiment may transmit a push signal having a narrow width and a high intensity.

Meanwhile, the ultrasound diagnosis apparatus 1000 according to an exemplary embodiment may additionally transmit a push signal to a shear wave generated inside an object, thereby superposing the given shear wave and a additionally generated shear wave. The ultrasound diagnosis apparatus 1000 may generate the superposed shear wave, thereby improving an SNR and accuracy of an elastography image.

A measurement error and system noise are present in all ultrasound systems. Thus, if a size of a signal that is to be measured by an ultrasound system is greater than the measurement error and the system noise, the SNR increases, and the accuracy of the elastography image is improved. However, a size of the shear wave that may be generated inside the object when the ultrasound diagnosis apparatus 1000 transmits a push signal is limited due to the stability of the object or limited resources of ultrasound systems.

The size of the shear wave that may be generated by transmitting the push signal once is limited, and, when the shear wave propagates inside the object, is attenuated, and thus it is difficult to acquire the SNR as high as is desired.

Therefore, the ultrasound diagnosis apparatus 1000 according to an exemplary embodiment may transmit an additional push signal based on the shear wave that propagates inside the body, thereby generating an additional shear wave. According to an exemplary embodiment, a previously propagating shear wave and the additionally-> ok generated shear wave are superposed, and thus the size of the shear wave that is to be observed is amplified.

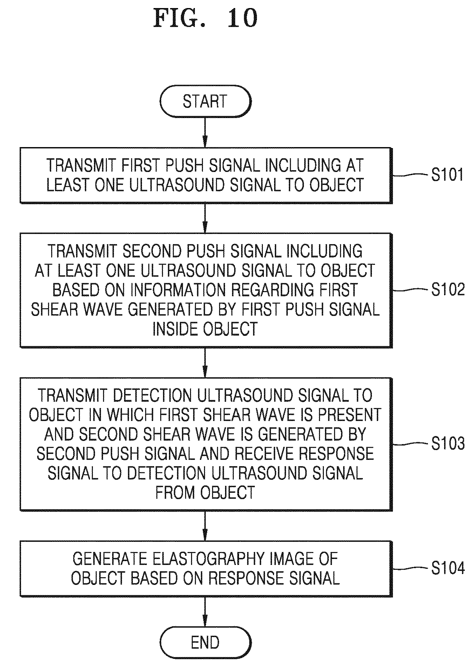

A method of additionally transmitting the push signal according to the propagation of the shear wave will now be described in more detail with reference to FIGS. 10 through 12 below.

FIG. 10 is a flowchart of a method of generating an elastography image according to an exemplary embodiment.

The method of generating the elastography image of FIG. 10 may be performed by the ultrasound diagnosis apparatus 1000 of FIG. 2. In more detail, operations S101 and S102 may be performed by the probe 1010 and the transmission unit 1021 of FIG. 2, operation S103 may be performed by the probe 1010 and the ultrasound transmission/reception unit 1020 of FIG. 2, and operation S104 may be performed by the image processing unit 1030 of FIG. 2. Thus, the description of FIG. 2 may be applied to the method of generating the elastography image of FIG. 10, and redundant descriptions are omitted.

In operation S101, the ultrasound diagnosis apparatus 1000 according to an exemplary embodiment may transmit a first push signal including at least one ultrasound signal to an object. The ultrasound diagnosis apparatus 1000 may generate a first shear wave inside the object by transmitting the first push signal to the object using at least one of a plurality of transducer elements.

For example, the ultrasound diagnosis apparatus 1000 may transmit the first push signal using all transducer elements included in the probe 1010 and using transducer elements included in at least one of a plurality of subnets constituting a plurality of transducer elements.

In operation S102, the ultrasound diagnosis apparatus 1000 according to an exemplary embodiment may transmit a second push signal including at least one ultrasound signal to the object based on information regarding the propagation of the first shear wave generated by the first push signal inside the object.

The ultrasound diagnosis apparatus 1000 may transmit the second push signal that generates a second shear wave that interferes constructively with the first shear wave. For example, the information regarding the propagation of the first shear wave may include a propagation velocity of the first shear wave or a location of a wave front of the first shear wave at a particular time. The ultrasound diagnosis apparatus 1000 according to an exemplary embodiment may induce a superposition of the first shear wave propagating inside the object and the second shear wave additionally generated by the second push signal by additionally transmitting the second push signal according to the traveling of the first shear wave.

As an example, the information regarding the propagation of the first shear wave may be previously stored information. The ultrasound diagnosis apparatus 1000 may previously store a propagation characteristic of a shear wave generated by transmitting a push signal inside the object. For example, the ultrasound diagnosis apparatus 1000 may previously store the propagation characteristic of the shear wave according to body information of the object or a body part. For example, the body information may include a sex, a height, a weight, an age, etc., and the body part may include a liver, an abdomen, breast, brain, etc. but the exemplary embodiments are not limited thereto.

As another example, the ultrasound diagnosis apparatus 1000 may transmit a detection ultrasound signal for acquiring the information regarding the propagation of the first shear wave after transmitting the first push signal before transmitting the second push single. The ultrasound diagnosis apparatus 1000 may receive a response signal of the transmitted detection ultrasound signal and extract the information regarding the propagation of the first shear wave from the response signal.

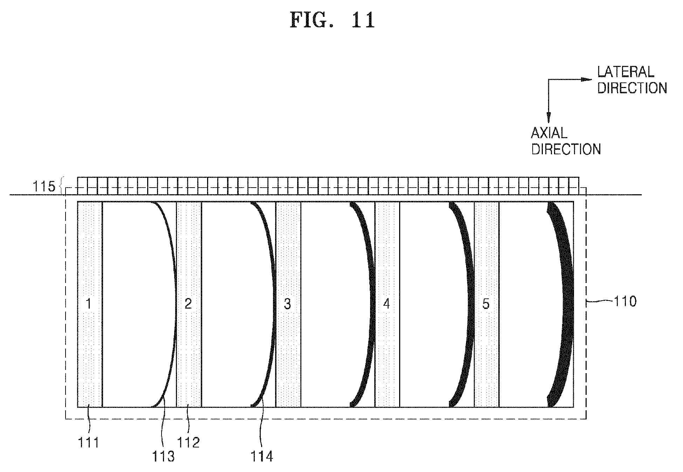

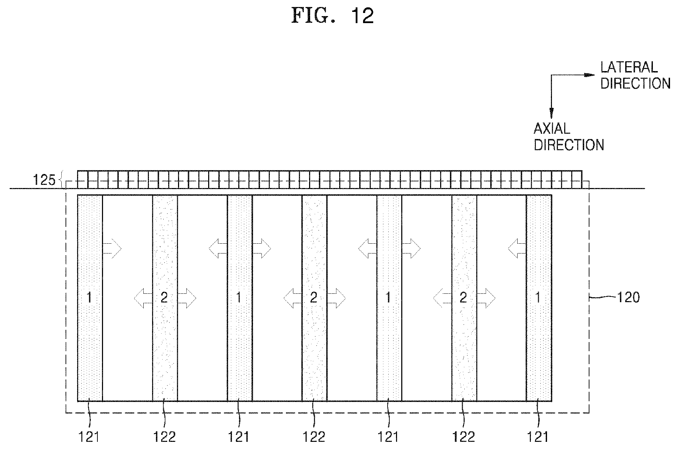

The ultrasound diagnosis apparatus 1000 according to an exemplary embodiment may adjust at least one of a location where the second push signal is transmitted and a time when the second push signal is transmitted based on the information regarding the propagation of the first shear wave. A method of transmitting the second push signal based on the information regarding the propagation of the first shear wave will be described in more detail with reference to FIGS. 11 and 12 later.

In operation S103, the ultrasound diagnosis apparatus 1000 according to an exemplary embodiment may transmit the detection ultrasound signal to the object in which the first shear wave is present and the second shear wave is generated by the second push signal. The ultrasound diagnosis apparatus 1000 may receive the response signal to the transmitted detection ultrasound signal from the object.

The ultrasound diagnosis apparatus 1000 may transmit the detection ultrasound signal to the object in which a shear wave is present in which the constructive interference occurs between the first shear wave and the second shear wave.

In operation S104, the ultrasound diagnosis apparatus 1000 according to an exemplary embodiment may generate the elastography image of the object based on the response signal.

For example, the ultrasound diagnosis apparatus 1000 may determine a propagation velocity of the shear wave in each region inside the object using the response signal. The ultrasound diagnosis apparatus 1000 may generate the elastography image using the propagation velocity of the shear wave.

The ultrasound diagnosis apparatus 1000 may generate the elastography image by mapping the propagation velocity of the shear wave or a shear modulus thereof to a color. According to an exemplary embodiment, the ultrasound diagnosis apparatus 1000 may display the elastography image overlapped on a B mode image.

FIGS. 11 and 12 are diagrams for describing adaptively transmitted push signals 111, 112, 121, and 122 according to an exemplary embodiment.

As an example, the ultrasound diagnosis apparatus 1000 according to an exemplary embodiment may adjust a location in which the second push signal 112 is transmitted based on information regarding the propagation of a first shear wave 113. As shown in FIG. 1, the ultrasound diagnosis apparatus 1000 may generate an additional shear wave by transmitting an additional push signal in consideration of a location of a wave front of a shear wave that propagates inside an object.

Firstly, the ultrasound diagnosis apparatus 1000 may generate the first shear wave 113 inside the object by transmitting the first push signal 111 to the object from at least one of a plurality of transducer elements 115.

Next, the ultrasound diagnosis apparatus 1000 may transmit the second push signal 112 to the object from at least one of the plurality of transducer elements 115 based on a location of a wave front of the first shear wave 113. The ultrasound diagnosis apparatus 1000 may transmit the second push signal 112 for generating a second shear wave that interferes constructively with the first shear wave 113 based on the location of the wave front of the first shear wave 113.

The ultrasound diagnosis apparatus 1000 may adaptively adjust the location in which the second push signal 112 is transmitted based on the information regarding the propagation of the first shear wave 113. The ultrasound diagnosis apparatus 1000 may determine which locations and how many transducer elements among the plurality of transducer elements 115 are used to transmit the second push signal 112 based on the adjusted location in which the second push signal 112 is transmitted.

As shown in FIG. 11, according to an exemplary embodiment, the first shear wave 113 that has been previously propagating and the additionally generated additional shear wave are superposed, and thus a shear wave 114 of which size is amplified is observed. The ultrasound diagnosis apparatus 1000 may sequentially transmit a plurality of push signals in a lateral direction of an ROI 110 inside the object, thereby generating an elastography image over the entire ROI 110. FIG. 11 illustrates a case where a push signal is transmitted five times while changing a transmission location of the push signal from left to right.

When the ultrasound diagnosis apparatus 1000 according to an exemplary embodiment transmits the plurality of push signals to have a consistent time interval t, the ultrasound diagnosis apparatus 1000 may determine a location where a next push signal is to be transmitted based on a propagation velocity of a shear wave generated by a previous push signal. A case where the ultrasound diagnosis apparatus 1000 transmits an i+1th push signal in consideration of a shear wave generated by an ith push signal is described as an example.

A distance interval D(i) between the ith push signal and the i+1th push signal may be acquired according to Equation 6 below. In Equation 6, Cs(i) denotes a propagation velocity of a shear wave generated by the ith push signal. The propagation velocity of the shear wave may be a predefined invariable value, a previously stored value according to body information of the object or a body part, or a value estimated through a previous measurement.

[Equation 6]

$$D(i) = Cs(i) \cdot t$$

As another example, the ultrasound diagnosis apparatus 1000 according to an exemplary embodiment may adjust a time when the second push signal 112 is transmitted based on the information regarding the propagation of the first shear wave 113. The ultrasound diagnosis apparatus 1000 may generate an additional shear wave by transmitting an additional push signal in consideration of a propagation velocity of a shear wave propagating inside the object. The ultrasound diagnosis apparatus 1000 may determine a time when the additional push signal is to be transmitted based on the propagation velocity of the shear wave generated by a push signal.

Firstly, the ultrasound diagnosis apparatus 1000 may generate the first shear wave 113 by transmitting the first push signal 111 to the object from at least one of the plurality of transducer elements 115.

Next, the ultrasound diagnosis apparatus 1000 may transmit the second push signal 112 to the object from at least one of the plurality of transducer elements 115 based on a propagation velocity of the first shear wave 113. The ultrasound diagnosis apparatus 1000 may adaptively adjust the time when the second push signal 112 is transmitted based on the propagation velocity of the first shear wave 113.

As shown in FIG. 12, the ultrasound diagnosis apparatus 1000 according to an exemplary embodiment may alternately apply comb push signal sets. A comb push signal set is configured as a plurality of push signals spaced apart from each other in the form of a comb. The ultrasound diagnosis apparatus 1000 may set two or more push signal sets in the form of the comb, transmit the comb push signal sets 121, and then determine an optimum transmission time for transmitting next comb push signal sets 122.

The ultrasound diagnosis apparatus 1000 may estimate a propagation velocity of a first shear wave generated by the comb push signal sets 121 and adaptively determine the transmission time of the comb push signal sets 122 based on the estimated propagation velocity.

As shown in FIG. 12, the ultrasound diagnosis apparatus 1000 may transmit the first comb push signal sets 121, estimate the propagation velocity of the shear wave generated by the first comb push signal sets 121, and transmit the second comb push signal sets 122 at a time determined on the basis of the estimated propagation velocity of the shear wave. The ultrasound diagnosis apparatus 1000 may adjust an optimum transmission time of a push signal based on the estimated propagation velocity of the shear wave, thereby amplifying a size of a shear wave continuously propagating in an entire ROI 120. An arrow of FIG. 12 indicates a propagation direction of each shear wave.

The ultrasound diagnosis apparatus 1000 according to an exemplary embodiment may transmit a plurality of push signals at invariable locations and determine a time when a next push signal is to be transmitted based on a propagation velocity of a shear wave generated by a previous push signal. A case where the ultrasound diagnosis apparatus 1000 transmits an i+1th push signal in consideration of a shear wave generated by an ith push signal is described as an example.

A transmission time interval t(i) between the ith push signal and the i+1th push signal may be acquired according to Equation 7 below.

[Equation 7]

$$t(i) = \frac{D(i)}{Cs(i)}$$

In Equation 7, D(i) denotes a distance in the transmission location between the ith push signal and the i+1th push signal. When the ultrasound diagnosis apparatus 1000 transmits the plurality of push signals at invariable locations, the distance D(i) in the transmission location between the ith push signal and the i+1th push signal may be obtained. For example, when push signals in the form of a comb are alternately transmitted as shown in FIG. 12, push signals included in next comb push signal sets are transmitted to a center location of two adjacent push signals included in previous comb push signal sets.

In Equation 7, Cs(i) denotes a propagation velocity of a shear wave generated by the ith push signal. The propagation velocity of the shear wave may be a predefined invariable value, a previously stored value according to body information of the object or a body part, or a value estimated through a previous measurement.

Meanwhile, a general ultrasound diagnosis apparatus may simultaneously drive all active transducer elements to transmit push signals to a specific location (for example, a specific point, line, or plane) during a predetermined period of time and simultaneously stop transmitting push signals in order to transmit a focused push signal or an unfocused push signal. The active transducer elements may mean transducer elements used to transmit a push signal.

An ultrasound diagnosis apparatus may use only a part of a plurality of transducer elements included in a probe or may use all of the plurality of transducer elements simultaneously to transmit an ultrasound signal. Thus, the active transducer elements may include entire of the plurality of transducer elements or part of the plurality of transducer elements included in the probe according to a type or a characteristic of a push signal that is transmitted.

A shape and a characteristic of a shear wave generated by a push signal may be determined by a beam profile of the push signal. For example, a center frequency of the shear wave, a bandwidth, etc. may be included in the characteristic of the shear wave. A requirement (for example, a minimum frame rate of a detection ultrasound signal transmitted and received to detect the shear wave) of the ultrasound diagnosis apparatus for detecting the shear wave generated inside the object may be passively determined enough to observe a progress of the generated shear wave.

In this regard, according to a general method of generating the shear wave by the push signal transmitted by simultaneously driving all active transducer elements, a limited condition for the ultrasound diagnosis apparatus required to detect the generated shear wave increases. The shape of the shear wave is also invariable, and thus an SNR of the shear wave that may be observed by transmitting the push signal once is also invariable.

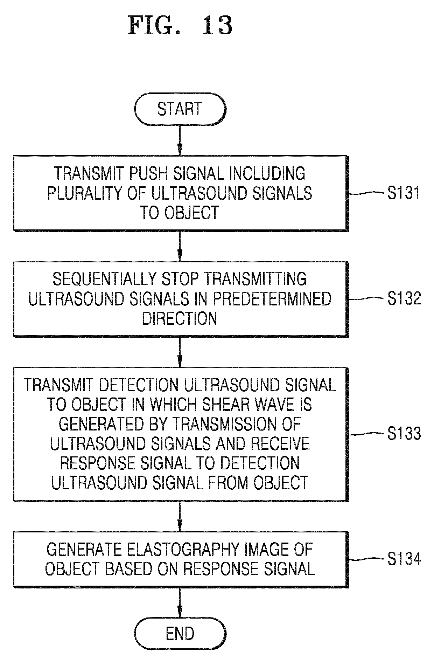

Therefore, the ultrasound diagnosis apparatus 1000 according to an exemplary embodiment may differently control driving of each transducer element over time, thereby adjusting the shape and the characteristic of the generated shear wave as desired. In more detail, the ultrasound diagnosis apparatus 1000 according to an exemplary embodiment may control on and off timing of each transducer element included in active transducer elements used to transmit the push signal, thereby adjusting the shape of the shear wave generated by the push signal. A method in which the ultrasound diagnosis apparatus 1000 according to an exemplary embodiment controls the shape and the characteristic of the shear wave will now be described in more detail with reference to FIGS. 13 through 16 below.

FIG. 13 is a flowchart of a method of generating an elastography image according to an exemplary embodiment.

The method of generating the elastography image of FIG. 13 may be performed by the ultrasound diagnosis apparatus 1000 of FIG. 2. In more detail, operations S131 and S132 may be performed by the probe 1010 and the transmission unit 1021 of FIG. 2, operation S133 may be performed by the probe 1010 and the ultrasound transmission/reception unit 1020 of FIG. 2, and operation S134 may be performed by the image processing unit 1030 of FIG. 2. Thus, the description of FIG. 2 may be applied to the method of generating the elastography image of FIG. 13, and redundant descriptions are omitted.

In operation S131, the ultrasound diagnosis apparatus 1000 according to an exemplary embodiment may transmit a push signal including a plurality of ultrasound signals to an object. The ultrasound diagnosis apparatus 1000 may transmit the push signal including the plurality of ultrasound signals using active transducer elements. The ultrasound diagnosis apparatus 1000 may use only a part of a plurality of transducer elements as active transducer elements to transmit the push signal.

The push signal transmitted by the ultrasound diagnosis apparatus 1000 according to an exemplary embodiment may be in the form of a push beam having directionality. The push signal transmitted by the ultrasound diagnosis apparatus 1000 to the object may be configured as the ultrasound signals that split a lateral width of the push signal into plural widths. The ultrasound diagnosis apparatus 1000 may be configured to transmit the ultrasound signals via apertures and may superpose the apertures, thereby driving all active transducer elements to transmit the push signal.

An "aperture" may mean a conceptual "opening" through which the ultrasound signals may be transmitted. The aperture may be a group of transducer elements that is collectively managed by the ultrasound diagnosis apparatus 1000 as a common group. For example, the aperture may include a group of transducer elements that may be physically distinguished from transducer elements included in adjacent apertures. However, adjacent apertures may not be necessarily distinguished physically.

As an example, two apertures may be disposed to be adjacent to each other on an array in which transducer elements are consecutively arranged. As another example, two apertures may be superposed to each other on the array. Thus, at least one transducer element may function as a part of two apertures. Locations and number of transducer elements included in the aperture and a physical size of the aperture may be dynamically defined in an arbitrarily way necessary for a specific application.

In operation S132, the ultrasound diagnosis apparatus 1000 according to an exemplary embodiment may sequentially stop transmitting the ultrasound signals in a predetermined direction. For example, the predetermined direction may include a lateral direction. The lateral direction, which is a direction in which the plurality of transducer elements that transmit the push signal are arranged, may be a direction perpendicular to a direction in which a push signal that is not steered is transmitted.

The ultrasound diagnosis apparatus 1000 may drive all of the active transducer elements to transmit the push signal and stop transmitting the ultrasound signals sequentially from an aperture transmitting a first ultrasound signal to an aperture transmitting an Nth ultrasound signal. One of two ultrasound signals transmitted from both ends in the lateral direction in which the active transducer elements are arranged may be the first ultrasound signal, and the other one may be the Nth ultrasound signal.

In other words, the ultrasound diagnosis apparatus 1000 according to an exemplary embodiment may transmit the push signal including the ultrasound signals arranged in the lateral direction, thereby generating a shear wave inside the object. In this regard, the ultrasound diagnosis apparatus 1000 according to an exemplary embodiment may start simultaneously transmitting the ultrasound signals via the apertures arranged in the lateral direction and then transmit them during different time cycles. A time cycle during which each ultrasound signal is transmitted may increase or decrease in the lateral direction. For example, provided that one of two ultrasound signals arranged in an end of the lateral direction of the push signal is the first ultrasound signal, the other one is the Nth ultrasound signal, and ultrasound signals disposed between the first and Nth ultrasound signals are second, third, ..., and N-1th ultrasound signals, according to an exemplary embodiment, the time cycle during which each ultrasound signal is transmitted may increase or decrease from the first ultrasound signal to the Nth ultrasound signal.

In operation S133, the ultrasound diagnosis apparatus 1000 according to an exemplary embodiment may transmit a detection ultrasound signal to the object in which a shear wave is generated by the transmission of the ultrasound signals, and receive a response signal to the detection ultrasound signal from the object.

In operation S134, the ultrasound diagnosis apparatus 1000 according to an exemplary embodiment may generate the elastography image of the object based on the response signal.

For example, the ultrasound diagnosis apparatus 1000 may determine a propagation velocity of the shear wave in each region inside the object using the response signal. The ultrasound diagnosis apparatus 1000 may generate the elastography image using the propagation velocity of the shear wave.

The ultrasound diagnosis apparatus 1000 may generate the elastography image by mapping the propagation velocity of the shear wave or a shear modulus thereof to a color. According to an exemplary embodiment, the ultrasound diagnosis apparatus 1000 may display the elastography image overlapped on a B mode image.

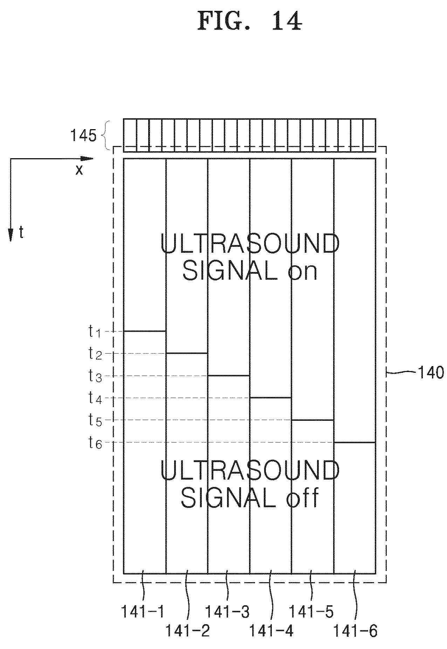

FIG. 14 is a diagram for describing a method of applying a push signal 140 so as to adjust a shape of a shear wave according to an exemplary embodiment.

As shown in FIG. 14, the push signal 140 transmitted according to an exemplary embodiment may be in the form of a push beam having directionality and may be configured as ultrasound beams that split a lateral beam width of the push beam into plural widths.

FIG. 14 illustrates the push signal 140 configured as 6 ultrasound signals 141-1, 141-2 141-3, 141-4, 141-5, and 141-6 that are transmitted through 6 apertures including a plurality of active transducer elements 145 but the exemplary embodiments are not limited to numbers of the apertures and the ultrasound beams of FIG. 14.

As shown in FIG. 14, the push signal 140 may be configured as the ultrasound signals 141-1, 141-2 141-3, 141-4, 141-5, and 141-6 that are arranged in a later direction (an x axis direction) in which the actived transducer elements 145 are arranged. In FIG. 14, an x axis is an axis of the lateral direction in which the actived transducer elements 145 are arranged, and a t axis is a time axis indicating a time when the ultrasound signals 141-1, 141-2 141-3, 141-4, 141-5, and 141-6 are transmitted from the actived transducer elements 145 and a time when the ultrasound signals 141-1, 141-2 141-3, 141-4, 141-5, and 141-6 stop being transmitted.

As shown in FIG. 14, the ultrasound diagnosis apparatus 1000 according to an exemplary embodiment may sequentially stop transmitting the ultrasound signals 141-1, 141-2 141-3, 141-4, 141-5, and 141-6 to an object from the actived transducer elements 145 in the lateral direction. The ultrasound diagnosis apparatus 1000 may stop transmitting the first ultrasound signal 141-1 at a time t1 via a first aperture, may stop transmitting the second ultrasound signal 141-2 at a time t2 via a second aperture, and may stop transmitting the third ultrasound signal 141-3 at a time t3 via a third aperture. The ultrasound diagnosis apparatus 1000 may sequentially stop transmitting the fourth, fifth, and sixth ultrasound signals 141-4, 141-5, and 141-6, respectively, at time t4, t5, and t6. In other words, a time cycle during which each of the ultrasound signals 141-1, 141-2 141-3, 141-4, 141-5, and 141-6 is transmitted may increase from the first ultrasound signal 141-1 to the sixth ultrasound signal 141-6.

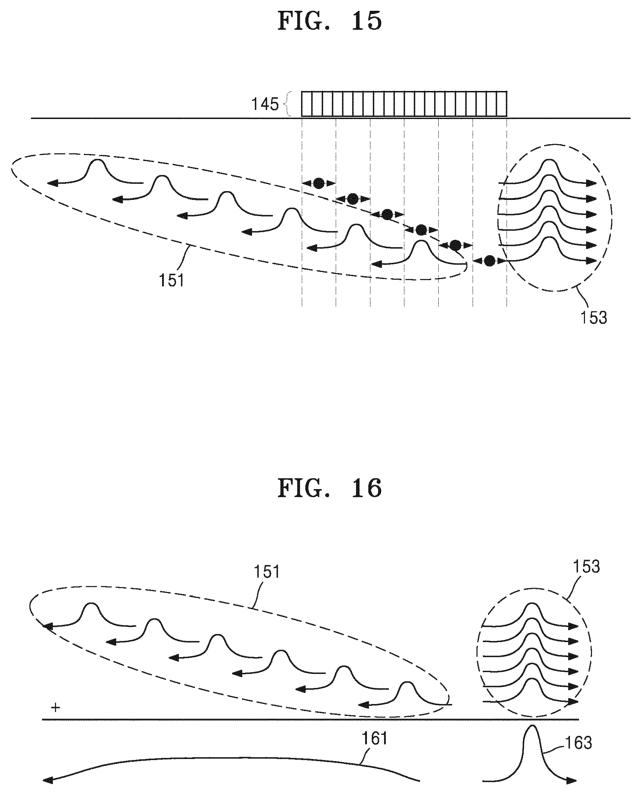

FIGS. 15 and 16 are diagrams for describing a method of adjusting shapes of shear waves 161 and 163 generated by applying time when ultrasound signals included in a push signal stop being transmitted as shown in FIG. 14.

As shown in FIG. 15, sub-shear waves generated by ultrasound signals due to time differences between the applied ultrasound signals may have time differences. Hereinafter, for convenience of description, a shear wave component generated by each ultrasound signal is a sub-shear wave.

The shear wave 161 configured as sub-shear waves 151 traveling in an opposite direction (hereinafter referred to as a "second direction") to a direction (a right direction of FIGS. 14 and 15)(hereinafter referred to as a "first direction") in which the ultrasound signals stop being transmitted may have a long wavelength. The shear wave 161 traveling in the second direction may have a low center frequency and a narrow bandwidth.

Meanwhile, the shear wave 163 configured as sub-shear waves 153 traveling in the direction (the first direction) in which the ultrasound signals stop being transmitted may have a short wavelength and a large amplitude due to the constructive interference of the sub-shear waves 153. The shear wave 163 traveling in the first direction may have a high center frequency and a wide bandwidth.

Therefore, as shown in FIG. 14, the ultrasound diagnosis apparatus 1000 according to an exemplary embodiment may sequentially stop transmitting ultrasound signals in a particular direction, and thus a shear wave propagating in the particular direction may have the large amplitude. Thus, an SNR of the shear wave propagating in the particular direction may be improved, thereby increasing accuracy of a generated elastography image.

Meanwhile, a shear wave, which propagates in an opposite direction to the particular direction that is the direction in which the ultrasound signals stop being transmitted, has a long wavelength, and thus the ultrasound diagnosis apparatus 1000 having a low frame rate may easily detect the shear wave. Thus, the ultrasound diagnosis apparatus 1000 according to an exemplary embodiment may actively control a shape and a characteristic of the generated shear wave, thereby increasing the accuracy of the elastography image, and generating a shear wave suitable for shear wave detection performance of the ultrasound diagnosis apparatus 1000.

Meanwhile, a high speed imaging system that transmits plane ultrasound waves transmits a very high frame rate of an ultrasound signal, and thus it is possible to detect a shear wave generated inside the object easily. However, an image generated using image data acquired at a high frame rate by the high speed imaging system has a bad resolution compared to a method of focusing ultrasound signals on various locations of the object and transmitting and receiving the ultrasound signals. Thus, a compound of a plurality of frames may be used. However, since the compound of the plurality of frames requires a very large amount of calculation, it is difficult for a scan line based ultrasound diagnosis apparatus having a low frame rate to implement the compound of the plurality of frames.

Meanwhile, a scan line based ultrasound diagnosis apparatus having a low frame rate capable of parallel beamforming may reduce the number of ultrasound signals to be transmitted and may apply Rx parallel beamforming to received ultrasound signals, and thus a method of increasing a pulse repetition frequency (PRF) of each scan line to a sufficiently high value by as much as high resolution may be used.

However, when parallel beamforming is used, since a calculation speed or a data size is in proportion to the number of parallel beamforming, an increase in the number of parallel beamforming is limited. In regard to image quality, compared to a general ultrasound diagnosis apparatus that transmits and receives ultrasound signals to all scan lines, since the number of ultrasound signals to be transmitted is small, image quality and contrast may be reduced.

The ultrasound diagnosis apparatus 1000 according to an exemplary embodiment proposes a method of detecting a shear wave in an ultrasound system (capable of performing low frame rate ultrasound imaging does not support transmission and reception of plane waves when detecting the shear wave generated inside an object. The ultrasound diagnosis apparatus 1000 according to an exemplary embodiment is a technology of generating an elastography image by combining temporally decimated shear wave detection signals acquired through several repetitive measurements.

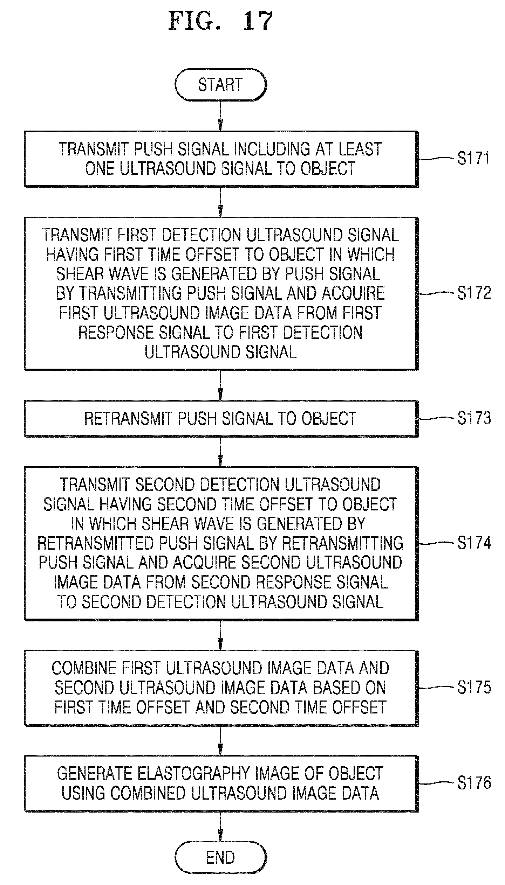

FIG. 17 is a flowchart of a method of generating an elastography image according to an exemplary embodiment.

The method of generating the elastography image of FIG. 17 may be performed by the ultrasound diagnosis apparatus 1000 of FIG. 2. In more detail, operations S171 and S173 may be performed by the probe 1010 and the transmission unit 1021 of FIG. 2, operations S172 and S174 may be performed by the probe 1010 and the ultrasound transmission/reception unit 1020 of FIG. 2, and operations S175 and S176 may be performed by the image processing unit 1030 of FIG. 2. Thus, the description of FIG. 2 may be applied to the method of generating the elastography image of FIG. 17, and redundant descriptions are omitted.

In operation S171, the ultrasound diagnosis apparatus 1000 according to an exemplary embodiment may transmit a push signal including at least one ultrasound signal to an object. The ultrasound diagnosis apparatus 1000 may generate a shear wave by transmitting the push signal to the object from a plurality of transducer elements.

In operation S172, the ultrasound diagnosis apparatus 1000 according to an exemplary embodiment may transmit a first detection ultrasound signal having a first time offset to the object in which a shear wave is generated by the push signal by transmitting the push signal. The ultrasound diagnosis apparatus 1000 may acquire first ultrasound image data from a first response signal to the first detection ultrasound signal.

In operation S173, the ultrasound diagnosis apparatus 1000 according to an exemplary embodiment may retransmit the push signal to the object from the plurality of transducer elements. The ultrasound diagnosis apparatus 1000 may retransmit the push signal that is the same as the push signal transmitted in operation S171, thereby generating the same shear wave as the shear wave generated by the push signal transmitted in operation S 171 inside the object.

In operation S174, the ultrasound diagnosis apparatus 1000 according to an exemplary embodiment may transmit a second detection ultrasound signal having a second time offset to the object in which the shear wave is generated by the retransmitted push signal by retransmitting the push signal. The ultrasound diagnosis apparatus 1000 may acquire second ultrasound image data from a second response signal to the second detection ultrasound signal.

The first time offset and the second time offset may be different from each other. A high speed ultrasound imaging system that transmits and receives plane ultrasound waves may support a high frame rate (for example, a frame rate higher than 1kHz), thereby acquiring ultrasound image data including many numbers of image frames enough to generate the elastography image. However, a low speed ultrasound imaging system that supports a low frame rate may acquire a decimated small number of image frames according to a difference in the supported frame rate, compared to the high speed ultrasound imaging system.

Therefore, the ultrasound diagnosis apparatus 1000 according to an exemplary embodiment may differentiate a time offset between the transmission of push signals and the transmission of detection ultrasound signals for detecting the shear wave and repeatedly receive response signals to the detection ultrasound signals. The ultrasound diagnosis apparatus 1000 may acquire a plurality of ultrasound image data sets from the repeatedly received response signals.

In operation S175, the ultrasound diagnosis apparatus 1000 according to an exemplary embodiment may combine the first ultrasound image data and the second ultrasound image data based on the first time offset and the second time offset.

The ultrasound diagnosis apparatus 1000 may temporally interleave the first ultrasound image data and the second ultrasound image data alternately, thereby generating combined ultrasound image data of the first ultrasound image data and the second ultrasound image data. The first ultrasound image data may be inserted into the combined ultrasound image data as ultrasound image frames corresponding to the first time offset. The second ultrasound image data may be inserted into the combined ultrasound image data as ultrasound image frames corresponding to the second time offset.

In operation S176, the ultrasound diagnosis apparatus 1000 according to an exemplary embodiment may generate the elastography image of the object using the combined ultrasound image data of operation S 175.

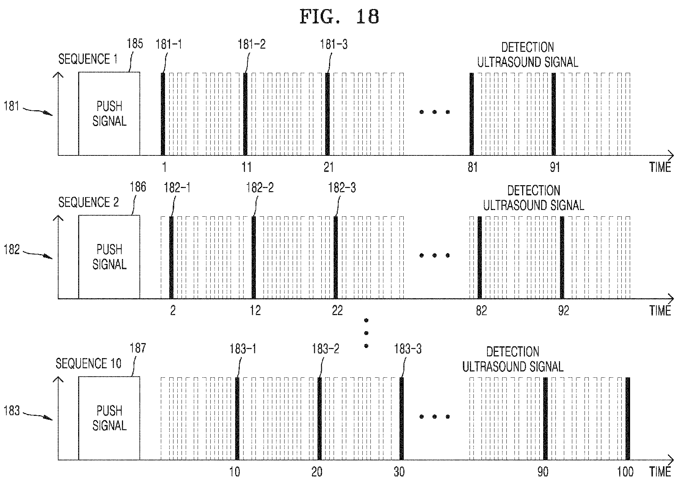

The ultrasound diagnosis apparatus 1000 may combine ultrasound image data sets corresponding to different time offsets, thereby acquiring entire ultrasound image data of a higher sampling rate. The ultrasound diagnosis apparatus 1000 may generate the elastography image using the entire ultrasound image data. Hereinafter, a method of generating the elastography image by combining temporally decimated ultrasound image data will now be described in more detail with reference to FIGS. 18 through 21.

FIG. 18 is a diagram for describing a method of acquiring temporally decimated scan line image data so as to detect a shear wave according to an exemplary embodiment. The ultrasound diagnosis apparatus 100 that supports a frame rate of 100 Hz is described as an example in FIG. 18 on the assumption that it is required to acquire ultrasound image data at a minimum frame rate of 1 kHz so as to detect the shear wave generated in an object.

As shown in FIG. 18, the ultrasound diagnosis apparatus 1000 according to an exemplary embodiment may transmit detection ultrasound signals 181-1, 181-2, 181-3, 182-1, 182-2, 182-3, 183-1, 183-2, and 183-3 for detecting the shear wave by differentiating time offsets by transmitting push signals 185, 186, and 187.

Since a PRF for each scan line is 100 Hz in the ultrasound diagnosis apparatus 1000 at the frame rate of 100 Hz, the ultrasound diagnosis apparatus 1000 may repeat sequences of transmission of push signals and transmission and reception of detection ultrasound signals for each scan line 10 times in order to detect the shear wave at the PRF (or a sampling rate) of 1 kHz.

The ultrasound diagnosis apparatus 1000 may repeat sequences of transmission of push signals and transmission and reception of detection ultrasound signals 10 times by increasing, by 1 ms, a time interval between a time when the transmission of the push signals ends and a time when the detection ultrasound signals are transmitted.

As shown in a graph 181 regarding a sequence 1, the ultrasound diagnosis apparatus 1000 may transmit the detection ultrasound signal 181-1 having a time offset of 1 ms to a scan line inside the object after transmitting the push signal 181. Since the ultrasound diagnosis apparatus 100 supports the frame rate of 100 Hz in an example of FIG. 18, the ultrasound diagnosis apparatus 100 may transmit the detection ultrasound signal 181-2 after 1/100 s from a time (1 ms) when the detection ultrasound signal 181-1 is transmitted and the detection ultrasound signal 181-3 for detecting the shear wave after 1/100 s from a time when the detection ultrasound signal 181-2 is transmitted.

As shown in a graph 182 regarding a sequence 2, the ultrasound diagnosis apparatus 1000 may transmit the detection ultrasound signal 182-1 having a time offset of 2 ms to a scan line inside the object after transmitting the push signal 186. Since the ultrasound diagnosis apparatus 100 supports the frame rate of 100 Hz in the example of FIG. 18, the ultrasound diagnosis apparatus 100 may transmit the detection ultrasound signal 182-2 after 1/100 s from a time (2 ms) when the detection ultrasound signal 182-1 is transmitted and the detection ultrasound signal 182-3 for detecting the shear wave after 1/100 s from a time when the detection ultrasound signal 182-2 is transmitted.

As shown in a graph 183 regarding a sequence 10, the ultrasound diagnosis apparatus 1000 may transmit the detection ultrasound signal 183-1 having a time offset of 3 ms to a scan line inside the object after transmitting the push signal 187. The ultrasound diagnosis apparatus 100 that supports the frame rate of 100 Hz may transmit the detection ultrasound signal 183-2 after 1/100 s from a time (3 ms) when the detection ultrasound signal 183-1 is transmitted and the detection ultrasound signal 183-3 for detecting the shear wave after 1/100 s from a time when the detection ultrasound signal 183-2 is transmitted.

As shown in FIG. 18, the ultrasound diagnosis apparatus 1000 according to an exemplary embodiment may repeatedly receive response signals to detection ultrasound signals having different time offsets and acquire a plurality of pieces of ultrasound image data from the repeatedly received response signals.

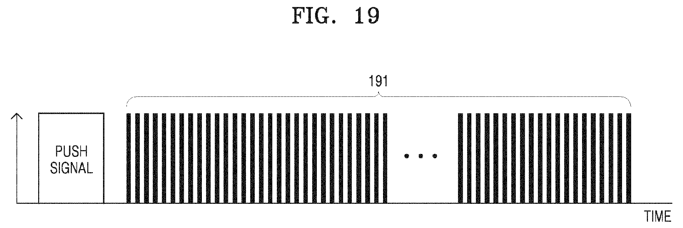

As shown in FIG. 19, the ultrasound diagnosis apparatus 1000 according to an exemplary embodiment may acquire entire ultrasound image data 191 of a high sampling rate by combining ultrasound image data corresponding to different time offsets. The ultrasound diagnosis apparatus 1000 may generate an elastography image using the entire ultrasound image data 191.

The ultrasound diagnosis apparatus 1000 may acquire ultrasound image data sets from response signals received in response to detection ultrasound signals and interleave the acquired ultrasound image data sets, thereby generating the entire ultrasound image data 191 having a PRF of 1 kHz. Ultrasound image data acquired through a kth sequence is kth, 10+kth, 20+kth, ..., 90+kth frames and is disposed in the entire ultrasound image data 191 when the ultrasound diagnosis apparatus 1000 interleaves the acquired ultrasound image data sets. The ultrasound diagnosis apparatus 1000 may generate the elastography image using the entire ultrasound image data 191 having the PRF of 1 kHz.

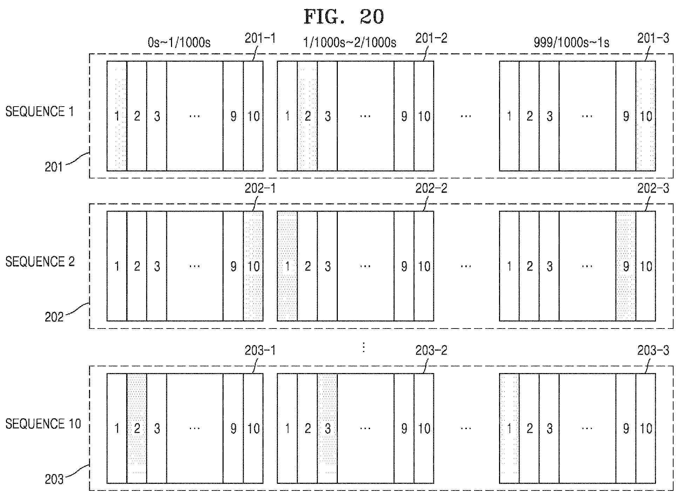

A method of acquiring temporally decimated scan line image data so as to detect a shear wave according to an exemplary embodiment will now be described in more detail with reference to FIG. 20 below.

A case where the ultrasound diagnosis apparatus 1000 supporting a frame rate of 100 Hz splits an ROI into 10 scan lines and acquires ultrasound image data 201, 201-1, 201-2, 201-3, 202, 202-1, 202-2, 202-3, 203, 203-1, 203-2, and 203-3 is described as an example with reference to FIG. 20. For convenience of description, a leftmost scan line is a first scan line, and scan lines right from the first scan line are sequentially second, third, ..., tenth scan lines in FIG. 20. It takes 10 ms for the ultrasound diagnosis apparatus 1000 supporting the frame rate of 100 Hz to acquire the ultrasound image data over the entire ROI.

When the ultrasound diagnosis apparatus 1000 transmits a detection ultrasound signal to the first scan line of the ROI as shown in FIG. 18, the ultrasound image data 201, 201-1, 201-2, 201-3, 202, 202-1, 202-2, 202-3, 203, 203-1, 203-2, and 203-3 is acquired in each sequence as shown in FIG. 20.

The ultrasound image data 201 of FIG. 20 is ultrasound image data acquired in a sequence 1 of FIG. 18. The ultrasound diagnosis apparatus 1000 may acquire the ultrasound image data 201-1 with respect to the first scan line at a time 0~1 ms after transmitting a push signal and acquire the ultrasound image data 201-2 with respect to the second scan line at a time 1~2 ms. The ultrasound diagnosis apparatus 1000 may repeatedly perform an operation of sequentially acquiring the ultrasound image data 201-1 and 201-2 with respect to each scan line. The ultrasound diagnosis apparatus 1000 may acquire the ultrasound image data 201-3 with respect to the tenth scan line at a time 999~1000 ms after transmitting the push signal.

The ultrasound image data 202 of FIG. 20 is ultrasound image data acquired in a sequence 2 of FIG. 18. The ultrasound diagnosis apparatus 1000 may transmit the detection ultrasound signal to the first scan line by applying a time offset different from a time offset in the sequence 1. The ultrasound diagnosis apparatus 1000 may transmit the detection ultrasound signal for detecting the shear wave to the first scan line in 2 ms after transmitting the push signal.

The ultrasound diagnosis apparatus 1000 may acquire the ultrasound image data 202-1 with respect to the tenth scan line at the time 0~1 ms after transmitting the push signal and acquire the ultrasound image data 202-2 with respect to the first scan line at the time 1~2 ms. The ultrasound diagnosis apparatus 1000 may repeatedly perform the operation of sequentially acquiring the ultrasound image data 202-1 and 202-2 with respect to each scan line. The ultrasound diagnosis apparatus 1000 may acquire the ultrasound image data 202-3 with respect to the ninth scan line at the time 999~1000 ms after transmitting the push signal.

The ultrasound image data 203 of FIG. 20 is ultrasound image data acquired in a sequence 10 of FIG. 18. The ultrasound diagnosis apparatus 1000 may transmit the detection ultrasound signal to the first scan line by applying a time offset different from time offsets in the sequences 1 through 9. The ultrasound diagnosis apparatus 1000 may transmit the detection ultrasound signal for detecting the shear wave to the first scan line in 9 ms after transmitting the push signal.

Therefore, the ultrasound diagnosis apparatus 1000 may acquire the ultrasound image data 203-1 with respect to the second scan line at the time 0~1 ms after transmitting the push signal and acquire the ultrasound image data 203-2 with respect to the third scan line at the time 1~2 ms. The ultrasound diagnosis apparatus 1000 may sequentially acquire the ultrasound image data 203-1 and 203-2 with respect to each scan line. The ultrasound diagnosis apparatus 1000 may acquire the ultrasound image data 203-3 with respect to the first scan line at the time 999~1000 ms after transmitting the push signal.

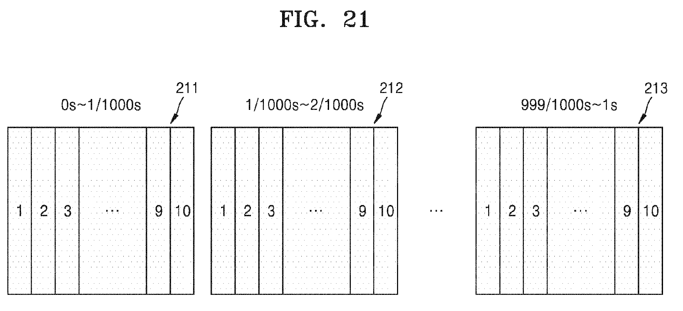

A method of generating an elastography image by combining temporally decimated scan line image data acquired in FIG. 20 will now be described in more detail with reference to FIG. 21 below.

As shown in FIG. 21, the ultrasound diagnosis apparatus 1000 may acquire ultrasound image data 211 over an entire ROI by combining the ultrasound image data 201-1, 202-1, ..., 203-1 acquired at a time 0~1 ms after transmitting a push signal in sequences 1 through 10. The ultrasound diagnosis apparatus 1000 may acquire ultrasound image data 212 over the entire ROI by combining the ultrasound image data 201-2, 202-2, ..., 203-2 acquired at a time 1~2 ms after transmitting the push signal in the sequences 1 through 10. The ultrasound diagnosis apparatus 1000 may acquire ultrasound image data 213 over the entire ROI by combining the ultrasound image data 201-3, 202-3, ..., 203-3 acquired at a time 999~1000 ms after transmitting the push signal in the sequences 1 through 10.

That is, as shown in FIG. 21, the ultrasound diagnosis apparatus 1000 according to an exemplary embodiment may combine ultrasound image data in which different time offsets are applied to scan lines, thereby acquiring entire ultrasound image data of a high sampling rate. The ultrasound diagnosis apparatus 1000 may generate the elastography image using the entire ultrasound image data.

A case where the ultrasound diagnosis apparatus 1000 supporting the frame rate of 100 Hz acquires ultrasound image data having the frame rate of 1 kHz is described as an example in FIGS. 18 through 21 but the exemplary embodiments are not limited thereto. When a PRF required to detect a shear wave is fe, and a PRF of the ultrasound diagnosis apparatus 1000 is fsys, ultrasound image data acquired through a kth sequence is an N(k)th frame and is disposed in the entire ultrasound image data. A location of the ultrasound image data acquired through the kth sequence in the entire ultrasound image data may be acquired according to Equations 8 and 9.

[Equation 8]

$$N(k) = m\frac{f\_e}{f\_sys} + k \qquad (m = 0, 1, \dots M)$$

[Equation 9]

$$M = F\frac{f_{sys}}{f_e} - 1$$

In Equation 9 above, F denotes a number of ultrasound image frames necessary for detecting the shear wave.

An exemplary embodiment of acquiring entire ultrasound image data of a high sampling rate by combining temporally decimated ultrasound image data is described with reference to FIGS. 17 through 2 above. Meanwhile, according to an exemplary embodiment, entire ultrasound image data of a high sampling rate may be acquired by combining spatially decimated ultrasound image data.

The ultrasound diagnosis apparatus 1000 according to an exemplary embodiment may generate the elastography image by combining spatially reduced shear wave detection ultrasound signals acquired through several repetitive measurements. A high speed imaging system that transmits plane ultrasound waves may simultaneously acquire ultrasound image data with respect to all scan lines. However, it takes relatively long time for a scan line based ultrasound diagnosis apparatus having a low frame rate to acquire ultrasound image data with respect to all scan lines.

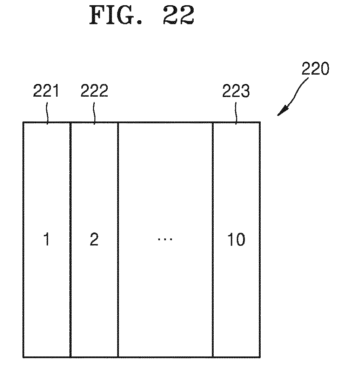

Therefore, as shown in FIG. 22, the ultrasound diagnosis apparatus 1000 according to an exemplary embodiment may individually perform a shear wave detection operation on each of scan lines 221, 222, ..., 223 of which lateral widths are reduced compared to a lateral width of an ROI 220. The ultrasound diagnosis apparatus 1000 may individually perform the shear wave detection operation on each of the scan lines 221, 222, ..., 223 to have a sufficiently high PRF with respect to each of the scan lines 221, 222, ..., 223 -> ok. The ultrasound diagnosis apparatus 1000 may spatially combine ultrasound image data acquired by repeatedly performing an operation of transmitting a push signal and detecting a shear wave, thereby acquiring the ultrasound image data of a desired resolution over the entire ROI 220.

For example, when the ultrasound diagnosis apparatus 1000 acquires images with respect to laterally adjacent scan lines, the ultrasound diagnosis apparatus 1000 may acquire the ultrasound image data over the entire ROI 220 by concatenating the acquired images. Alternatively, when the ultrasound diagnosis apparatus 1000 acquires images with respect to scan lines spaced by a predetermined distance, the ultrasound diagnosis apparatus 1000 may acquire the ultrasound image data over the entire ROI 220 by interleaving the acquired images.

The ultrasound diagnosis apparatus 1000 according to an exemplary embodiment may acquire partial ultrasound image data at high frame rate and resolution with respect to a part of the ROI 220 using limited resources of the ultrasound diagnosis apparatus 1000 such as an amount of calculation, a memory capacity, etc. The ultrasound diagnosis apparatus 1000 may acquire the ultrasound image data over the entire ROI 220 by concatenating the partial ultrasound image data at high frame rate and resolution.

The ultrasound diagnosis apparatus 1000 that acquires the partial ultrasound image data with respect to the part of the ROI 220 has a small image region that is to be processed at a time, thereby acquiring the ultrasound image data robustly to a movement of an object. The ultrasound diagnosis apparatus 1000 acquires the partial ultrasound image data with respect to only the part of the ROI 220, and thus the ultrasound diagnosis apparatus 1000 may be less influenced by attenuation of the shear wave when the shear wave propagates into the entire ROI 220.

Meanwhile, the ultrasound diagnosis apparatus 1000 according to an exemplary embodiment may maintain a PRF of each scan line by reducing an axial length of the ROI 220 while securing lateral width and resolution of the ROI 220.

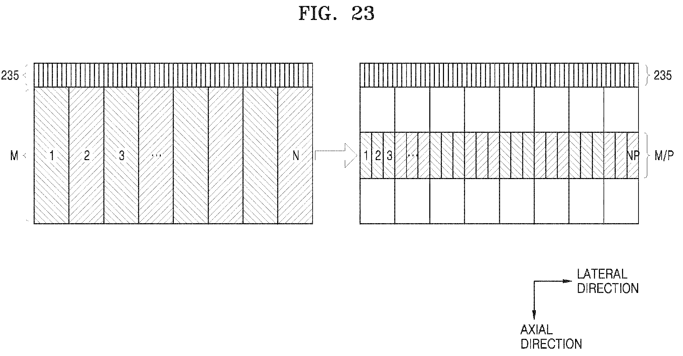

As shown in FIG. 23, if a number of scan lines that may be acquired by the ultrasound diagnosis apparatus 1000 at a minimum frame rate (for example, a frame rate of 1 kHz) for detecting a shear wave is N, and a number of samples constituting data with respect to one scan line is M, the ultrasound diagnosis apparatus 1000 according to an exemplary embodiment may process on PN scan lines by reducing the number of the samples by M/P. The ultrasound diagnosis apparatus 1000 may secure an increased number of scan lines by applying, P times, Rx parallel beamforming on an ultrasound signal transmitted once. The ultrasound diagnosis apparatus 1000 may increase a lateral resolution of a generated elastography image or may set a wide lateral width of an ROI by securing the increased number of scan lines. In this regard, since the total number of samples acquired by the ultrasound diagnosis apparatus 1000 by Rx parallel beamforming on the ultrasound signal is the same, an additional calculation device or memory for Rx parallel beamforming is not necessary, and an amount of transmitted data is the same.

Meanwhile, a general ultrasound diagnosis apparatus supporting parallel beamforming may transmit an ultrasound signal once and acquire ultrasound image data with respect to a plurality of scan lines from a response signal to the transmitted ultrasound signal. According to parallel beamforming, ultrasound image data with respect to an entire ROI may be acquired faster than an ultrasound signal is transmitted and received with respect to each scan line. However, ultrasound image data acquired using parallel beamforming has a low image quality since a small number of ultrasound signals are transmitted.

Therefore, the ultrasound diagnosis apparatus supporting parallel beamforming needs to individually perform operations of acquiring the elastography image and a B mode image so as to display the elastography image on the B mode image.

That is, since it is more important to acquire ultrasound image data at a high PRF than acquire the ultrasound image data of a high resolution so as to generate the elastography image, the ultrasound diagnosis apparatus may acquire the ultrasound image data by applying parallel beamforming, whereas, since it is more important to acquire the ultrasound image data of the high resolution than acquire the ultrasound image data at the high PRF so as to generate the B mode image, the ultrasound diagnosis apparatus may acquire the ultrasound image data by transmitting and receiving an ultrasound signal to each scan line without applying parallel beamforming.

Therefore, if an operation of transmitting and receiving the ultrasound signal so as to acquire the elastography image and an operation of transmitting and receiving the ultrasound signal so as to acquire the B mode image are individually performed, there is a problem that an overall processing speed of the ultrasound diagnosis apparatus is very low.

The ultrasound diagnosis apparatus 1000 according to an exemplary embodiment may simultaneously perform the B mode image and the elastography image through Rx parallel beamforming, thereby maintaining a high frame rate.

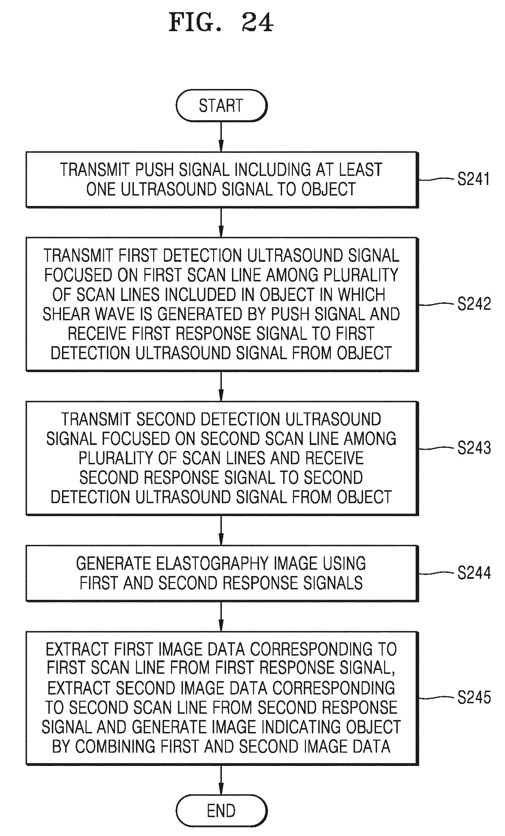

FIG. 24 is a flowchart of a method of generating an elastography image according to an exemplary embodiment.

The method of generating the elastography image of FIG. 24 may be performed by the ultrasound diagnosis apparatus 1000 of FIG. 2. In more detail, operation S241 may be performed by the probe 1010 and the transmission unit 1021 of FIG. 2, operations S242 and S243 may be performed by the probe 1010 and the ultrasound transmission/reception unit 1020 of FIG. 2, and operations S244 and S245 may be performed by the image processing unit 1030 of FIG. 2. Thus, the description of FIG. 2 may be applied to the method of generating the elastography image of FIG. 24, and redundant descriptions are omitted.

In operation S241, the ultrasound diagnosis apparatus 1000 according to an exemplary embodiment may transmit a push signal including at least one ultrasound signal to an object. The ultrasound diagnosis apparatus 1000 may generate the elastography image by transmitting the push signal to the object from a plurality of transducer elements.

In operation S242, the ultrasound diagnosis apparatus 1000 according to an exemplary embodiment may transmit a first detection ultrasound signal focused on a first scan line among a plurality of scan lines included in the object in which a shear wave is generated by the push signal. The ultrasound diagnosis apparatus 1000 may receive a first response signal to the first detection ultrasound signal from the object.

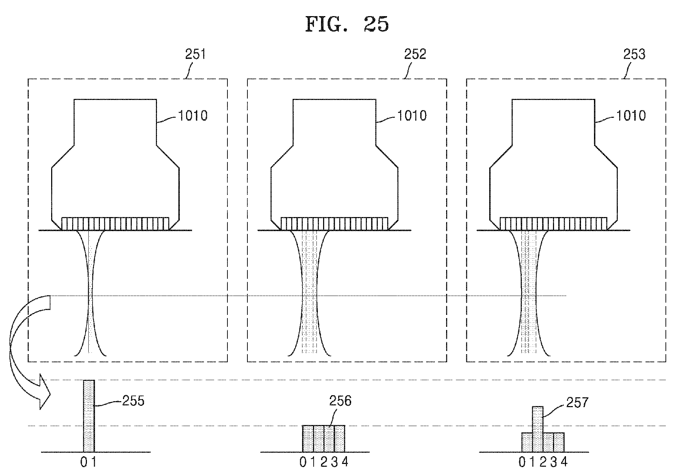

FIG. 25 is a diagram for describing a method of transmitting a detection ultrasound signal focused on a transmission reference scan line according to an exemplary embodiment.

Referring to FIG. 25, an image 251 shows that the probe 1010 included in an ultrasound diagnosis apparatus that does not support parallel beamforming transmits the detection ultrasound signal focused on one scan line. As shown in a graph 255, energy may be concentrated on one scan line.

An image 252 shows that the probe 1010 included in an ultrasound diagnosis apparatus that supports parallel beamforming transmits the detection ultrasound signal to a plurality of scan lines. As shown in a graph 256, ultrasound energy may be concentrated on the plurality of scan lines.

A limited amount of energy of the detection ultrasound signal may be transmitted by the ultrasound diagnosis apparatus 1000 to the object. Thus, as shown in the graphs 255 and 256, an average energy of the detection ultrasound signal transmitted to the plurality of scan lines is lower than an average energy of the detection ultrasound signal transmitted by being focused on one scan line. The ultrasound diagnosis apparatus 1000 may apply Rx parallel beamforming to a response signal received in response to the detection ultrasound signal, thereby acquiring ultrasound image data with respect to the plurality of scan lines by transmission and reception of the ultrasound signal once.

An image 253 shows that the probe 1010 included in the ultrasound diagnosis apparatus 1000 according to an exemplary embodiment transmits, to a plurality of scan lines, the detection ultrasound signal that is more focused on one of the plurality of scan lines than the other scan lines. When the ultrasound diagnosis apparatus 1000 transmits the detection ultrasound signal that is focused on one of the plurality of scan lines, the scan line on which the detection ultrasound signal is focused is the transmission reference scan line. To focus the detection ultrasound signal on one of the plurality of scan lines means to transmit the detection ultrasound signal whose energy is more concentrated on the transmission reference scan line than the other scan lines.

As shown in a graph 257, the ultrasound diagnosis apparatus 1000 may transmit the ultrasound signal to the plurality of scan lines. The ultrasound diagnosis apparatus 1000 may transmit the detection ultrasound signal of which energy is more concentrated on one scan line than the other scan lines. The ultrasound diagnosis apparatus 1000 may apply Rx parallel beamforming to a response signal received in response to the detection ultrasound signal, thereby acquiring ultrasound image data with respect to the plurality of scan lines by transmission and reception of the ultrasound signal once.

In operation S243, the ultrasound diagnosis apparatus 1000 according to an exemplary embodiment may transmit a second detection ultrasound signal focused on a second scan line among the plurality of scan lines. The ultrasound diagnosis apparatus 1000 may receive a second response signal to the second detection ultrasound signal from the object.

The ultrasound diagnosis apparatus 1000 according to an exemplary embodiment may transmit and receive the ultrasound signal with respect to the plurality of scan lines while acquiring ultrasound image data by shifting a location on which the ultrasound signal is focused for each frame.

In operation S244, the ultrasound diagnosis apparatus 1000 according to an exemplary embodiment may generate the elastography image using the first and second response signals.

The ultrasound diagnosis apparatus 1000 may apply Rx parallel beamforming to the first and second response signals, thereby acquiring ultrasound image frames with respect to the plurality of scan lines. The ultrasound diagnosis apparatus 1000 may generate the elastography image using the acquired ultrasound image frames.

In operation S245, the ultrasound diagnosis apparatus 1000 according to an exemplary embodiment may extract first image data corresponding to the first scan line from the first response signal. The ultrasound diagnosis apparatus 1000 may extract second image data corresponding to the second scan line from the second response signal. The ultrasound diagnosis apparatus 1000 may generate an image indicating the object by combining the first and second image data. For example, the image indicating the object may be the B mode image that expresses a tissue characteristic of a cross-section of the object in brightness.

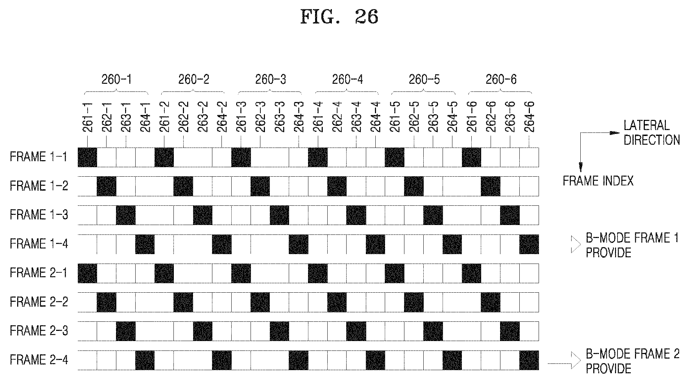

FIG. 26 is a diagram for describing a method of generating an elastography image and a B mode image by shifting a transmission reference scan line of a detection ultrasound signal, acquiring image data, and combining the acquired image data according to an exemplary embodiment.

A case where an ultrasound image data is acquired by splitting an ROI inside an object into 24 scan lines is described as an example in FIG. 26 but the exemplary embodiments are not limited thereto. A case where the ultrasound diagnosis apparatus 1000 performs fourfold Rx parallel beamforming is described as an example in FIG. 26 but the exemplary embodiments are not limited thereto.

As shown in FIG. 26, the ultrasound diagnosis apparatus 1000 may transmit an ultrasound signal focused on a first scan line 261 among a plurality of scan lines 260. The ultrasound diagnosis apparatus 1000 may receive a response signal to the ultrasound signal from an object. The ultrasound diagnosis apparatus 1000 may apply fourfold Rx parallel beamforming to the received response signal, thereby generating one sheet of an elastography image frame 1-1.

To generate a next elastography image frame 1-2, the ultrasound diagnosis apparatus 1000 may transmit an ultrasound signal focused on a second scan line 262 among the plurality of scan lines 260. The ultrasound diagnosis apparatus 1000 may receive a response signal to the ultrasound signal from the object. The ultrasound diagnosis apparatus 1000 may apply fourfold Rx parallel beamforming to the received response signal, thereby generating one sheet of the elastography image frame 1-2.

To generate a next elastography image frame 1-3, the ultrasound diagnosis apparatus 1000 may transmit an ultrasound signal focused on a third scan line 263 among the plurality of scan lines 260. To generate a next elastography image frame 1-4, the ultrasound diagnosis apparatus 1000 may transmit an ultrasound signal focused on a fourth scan line 264 among the plurality of scan lines 260.

The ultrasound diagnosis apparatus 1000 according to an exemplary embodiment may focus an ultrasound signal on all the scan lines 260 by shifting a scan line on which the ultrasound signal is focused, thereby acquiring ultrasound image data. -> ok That is, one sheet of a B mode image frame 1 may be generated per four sheets of elastography image frames 1-1, 1-2, 1-3, and 1-4 as shown in FIG. 26.

The ultrasound diagnosis apparatus 1000 may extract the image data corresponding to each of the scan lines 260 from the ultrasound image data acquired by transmitting the detection ultrasound signal several times. That is, referring to FIG. 26, the ultrasound diagnosis apparatus 1000 may extract the image data corresponding to the first scan line 261 from the elastography image frame 1-1 and the image data corresponding to the second scan line 262 from the elastography image frame 1-2. Thus, the ultrasound diagnosis apparatus 1000 may extract the image data corresponding to all the scan lines 260 from the four sheets of elastography image frames 1-1, 1-2, 1-3, and 1-4. The ultrasound diagnosis apparatus 1000 may combine the extracted image data, thereby generating the B mode image over an entire ROI.

Therefore, the ultrasound diagnosis apparatus 1000 according to an exemplary embodiment may simultaneously perform an operation of transmitting and receiving the ultrasound signal for generating the elastography image and an operation of transmitting and receiving the ultrasound signal for generating the B mode image, thereby increasing a processing speed of the ultrasound diagnosis apparatus 1000.

Meanwhile, the ultrasound diagnosis apparatus 1000 according to an exemplary embodiment may include more elements than those shown in FIG. 3.

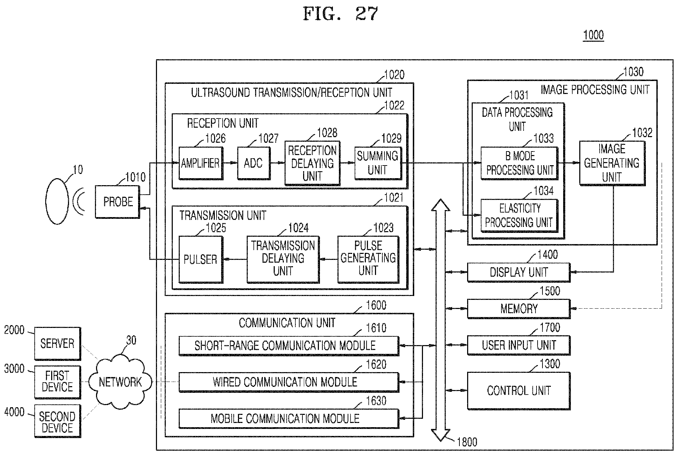

For example, as shown in FIG. 27, the ultrasound diagnosis apparatus 1000 according to an exemplary embodiment may further include a control unit 1300, a display unit 1400, a memory 1500, and a communication unit 1600, and a user input unit 1700. Various elements included in the ultrasound diagnosis apparatus 1000 may be connected to each other via a bus 1800.

Hereinafter, the above-described elements will be sequentially described in detail.

The probe 1010, the ultrasound transmission/reception unit 1020, and the image processing unit 1030 according to an exemplary embodiment may acquire ultrasound image data with respect to the object 10. The ultrasound image data according to an exemplary embodiment may be 2D ultrasound image data or 3D ultrasound image data with respect to the object 10.

The transmission unit 1021 included in the ultrasound transmission/reception unit 1020 according to an exemplary embodiment may include a pulse generating unit 1023, a transmission delaying unit 1024, and a pulser 1025 as shown in FIG. 27.

The transmission unit 1021 may supply a driving signal to the probe 1010. The pulse generating unit 1023 may generate pulses for forming transmission ultrasound waves based on a PRF. The transmission delaying unit 1024 may apply a delay time for determining transmission directionality to the pulses. Pulses to which a delay time is applied may correspond to a plurality of piezoelectric vibrators included in the probe 1010, respectively. The pulser 1025 may apply a driving signal (or a driving pulse) to the probe 1010 as a timing corresponding to each pulse to which a delay time is applied.

The reception unit 1022 included in the ultrasound transmission/reception unit 1020 according to an exemplary embodiment may include an amplifier 1026, an analog-digital converter (ADC) 1027, a reception delaying unit 1028, and a summing unit 1029 as shown in FIG. 27.

The reception unit 1022 may process a response signal received from the probe 1010 and generate ultrasound data. The amplifier 1026 may amplify the response signal in each channel. The ADC 1027 may analog-to-digital convert the amplified response signal. The reception delaying unit 1028 may apply a delay time for determining reception directionality to the digital-converted response signal. The summing unit 1029 may generate ultrasound image data by summing the response signals processed by the reception delaying unit 1028.

The probe 1010 according to an exemplary embodiment may wholly or partly include the elements included in the transmission unit 1021 and the reception unit 1022 of FIG. 27 and may perform wholly or partly functions performed by the unit 1021 and the reception unit 1022.

The image processing unit 1030 may generate an ultrasound image by scan-converting ultrasound data generated by the ultrasound transmission/reception unit 1020 and displays the ultrasound image. Meanwhile, an ultrasound image may include not only a grayscale ultrasound image obtained by scanning an object in an amplitude (A) mode, a brightness (B) mode, and a motion (M) mode, but also a blood flow Doppler image showing blood flow (aka a color Doppler image), a tissue Doppler image showing tissue movement, and a spectral Doppler image showing moving speed of an object as a waveform.

A B mode processing unit 1033 in a data processor 1031 may extract B mode components from ultrasound data and processes the B mode components. An image generating unit 1032 may generate an ultrasound image indicating signal intensities as brightness based on the extracted B mode components.

Similarly, the elasticity processing unit 1034 in a data processor 1031 may extract a shear-wave speed component (for example, a shear modulus) from elasticity data to process the shear-wave speed. The image generating unit 1032 may generate an elastography image in which the shear-wave speed is expressed as a color, on the basis of the shear-wave speed component (for example, the shear modulus) extracted by the elasticity processing unit 1034.

A Doppler processing unit (not shown) may extract Doppler components from ultrasound data. The image generating unit 1032 may generate a Doppler image indicating movement of an object as colors or waveforms based on the extracted Doppler components.

The image generating unit 1032 according to an exemplary embodiment may generate a 3D ultrasound image via volume-rendering of volume data and may also generate an elastography image which gives a visual representation of deformation of the object 10 due to a pressure.

Furthermore, the image generating unit 1032 may display various pieces of additional information in an ultrasound image by using texts and graphics. For example, the image generating unit 1032 may add at least one annotation, associated with all or a portion of an ultrasound image, to the ultrasound image. That is, the image generating unit 1032 may analyze the ultrasound image, and recommend the at least one annotation associated with all or a portion of an ultrasound image, based on a result of the analysis. Also, the image generating unit 1032 may add additional information, corresponding to an ROI selected by a user, to the ultrasound image.

Meanwhile, the image processing unit 1030 may extract an ROI from the ultrasound image by using an image processing algorithm. For example, the image processing unit 1030 may extract an ROI from an elastography image on the basis of a shear wave. In this case, the image processing unit 1030 may add a color, a pattern, or a border to the ROI.

The user input unit 1700 may denote a device that allows a user (for example, a sonographer) to input data used to control the ultrasound diagnosis apparatus 1000. For example, the user input unit 1700 may include a keypad, a dome switch, a touch pad (for example, of a contact capacitive type, a press resistive type, an infrared sensing type, a surface ultrasonic conductive type, an integration tension measurement type, or a piezo effect type), a jog wheel, and a jog switch, but is not limited thereto. For example, the user input unit 1700 may further include various other input devices including an electrocardiogram measuring module, a respiration measuring module, a voice recognition sensor, a gesture recognition sensor, a fingerprint recognition sensor, an iris recognition sensor, a depth sensor, a distance sensor, etc.

According to an exemplary embodiment, the user input unit 1700 may detect a proximity touch in addition to a real touch. The user input unit 1700 may detect a touch input (for example, touch & hold, tap, double taps, flick, or the like) for the ultrasound image. Also, the user input unit 1700 may detect a drag input from a position at which a touch input is detected. In addition, the user input unit 1700 may detect multi touch inputs (for example, pinches) for at least one two positions included in the ultrasound image.

According to an exemplary embodiment, the user input unit 1700 may receive EOI information from the user. For example, the user input unit 1700 may receive a range of a shear modulus as the EOI information. The user input unit 1700 may receive a central shear modulus and an application range as the EOI information. The user input unit 1200 may receive selection of an EOI range from an elasticity range list including a plurality of elasticity ranges.

According to an exemplary embodiment, the user input unit 1700 may receive information about an SOI from the user. The user input unit 1700 may receive a boundary deletion request for at least one TOI of a plurality of TOIs corresponding to the EOI information. According to an exemplary embodiment, the user input unit 1700 may receive an input that changes the EOI information.

The control unit 1300 may control an overall operation of the ultrasound diagnosis apparatus 1000. For example, the control unit 1300 may overall control the probe 1010, the ultrasound transmission/reception unit 1020, the image processing unit 1030, the user input unit 1700, the display unit 1400, the memory 1500, and the communication unit 1600.

The display unit 1400 may display and output information obtained through processing by the ultrasound diagnosis apparatus 1000. For example, the display unit 1400 may display the ultrasound image, or display a user interface (UI) or a GUI relating to a control panel.

The display unit 1400 may display an elastography image that is acquired by using a shear wave. In this case, the display unit 1400 may display the elastography image to transducer element on a B mode image. The display unit may mark a TOI on the elastography image. For example, the display unit 1400 may mark a boundary line on the TOI. The display unit 1400 may provide measurement information about a TOI. When a plurality of TOIs are detected, the display unit may provide measurement information corresponding to each of the plurality of TOIs.

When the display unit 1400 is configured with a touch screen in which a touch pad forms a layer structure, the display unit 1400 may be used as an input device in addition to an output device. The display unit 1400 may include at least one of a liquid crystal display (LCD), a thin film transistor-liquid crystal display (TFT-LCD), an organic light-emitting diode (OLED) display, a flexible display, a 3D display, and an electrophoretic display. The ultrasound apparatus 1000 may include two or more the display units 1200 depending on an implementation type of the ultrasound apparatus 1000.

The memory 1500 may store a program for processing of the control unit 1300, and store input/output data (for example, ultrasound image data, elasticity data, information about an ROI, an elasticity range list, examinee information, probe information, a body marker, additional information, etc.).

The memory 1500 may include at least one type of storage medium of a flash memory, a hard disk, a multimedia micro card, a card type memory (a secure digital (SD) card, an extreme digital (XD) card, or the like), a random access memory (RAM), a static random access memory (SRAM), a read-only memory (ROM), an electrically erasable programmable read-only memory (EEPROM), and a programmable read-only memory (PROM). Also, the ultrasound apparatus 1000 may use web storage or a cloud server which performs a storage function of the memory 1500 on the Web.

The communication unit 1600 may include one or more elements that enable communication between the ultrasound diagnosis apparatus 1000 and a server 2000, between the ultrasound diagnosis apparatus 1000 and a first device 3000, and between the ultrasound diagnosis apparatus 1000 and a second device 4000. For example, the communication unit 1600 may include a short-range communication module 1610, a wired communication module 1620, and a mobile communication module 1630.

The short-range communication module 1610 may refer to a module for close-distance communication within a predetermined distance. Examples of close-distance communication techniques according to an embodiment may include wireless LAN, Wi-Fi, Bluetooth, Zigbee, Wi-Fi Direct (WFD), ultra wideband (UWB), infrared data association (IrDA), Bluetooth Low Energy (BLE), and near field communication (NFC). However, the embodiments are not limited thereto.

The wired communication module 1620 refers to a module for communication using electric signals or optical signals. Examples of wired communication techniques according to an embodiment may include a pair cable, a coaxial cable, an optical fiber cable, and an Ethernet cable.

The mobile communication module 1630 transmits and receives wireless signals with at least one of a base station, external devices (for example, the first device 3000 and the second device 4000), and the server 2000 over a mobile communication network. Here, the wireless signals may include voice call signals, video call signals, or various types of data for transmission and reception of text/multimedia messages.

The communication unit 1600 is connected by wire or wirelessly to a network 30 and communicates with an external device or a server. The communication unit 1600 may exchange data with a hospital server or another medical device in a hospital that is connected with a picture archiving and communications system (PACS). Furthermore, the communication unit 1600 may perform data communication according to the digital imaging and communications in medicine (DICOM) standard.

The communication unit 1600 may transmit and receive data related to diagnosis of an object, e.g., an ultrasound image, ultrasound data, and Doppler data of the object, via the network 30 and may also transmit and receive medical images obtained via other medical devices, e.g., a computerized tomography (CT) image, a magnetic resonance (MR) image, and an X-ray image. Furthermore, the communication unit 1600 may receive information related to diagnosis history or treatment schedule of a patient from a server and utilize the information for diagnosing the patient.

One or more of the above embodiments may be implemented as computer readable codes in a non-transitory computer readable recording medium. The non-transitory computer readable recording medium may include a program instruction, a local data file, a local data structure, or a combination thereof. The non-transitory computer readable recording medium may be specific to exemplary embodiments or commonly known to those of ordinary skill in computer software. The non-transitory computer readable recording medium includes all types of recordable media in which computer readable data are stored. Examples of the non-transitory computer readable recording medium include a magnetic medium, such as a hard disk, a floppy disk and a magnetic tape, an optical medium, such as a CD-ROM and a DVD, a magneto-optical medium, such as a floptical disk, and a hardware memory, such as a ROM, a RAM and a flash memory, specifically configured to store and execute program instructions. Furthermore, the non-transitory computer readable recording medium may be implemented in the form of a transmission medium, such as light, wire, or waveguide, to transmit signals which designate program instructions, local data structures and the like. Examples of the program instructions include machine code, which is generated by a compiler, and a high level language, which is executed by a computer using an interpreter and so on.

It should be understood that exemplary embodiments described herein should be considered in a descriptive sense only and not for purposes of limitation. Descriptions of features or aspects within each exemplary embodiment should typically be considered as available for other similar features or aspects in other exemplary embodiments.

While one or more exemplary embodiments have been described with reference to the figures, it will be understood by those of ordinary skill in the art that various changes in form and details may be made therein without departing from the spirit and scope as defined by the following claims.

**Claims**

1. A method of generating an elastography image, the method comprising:

   transmitting unfocused push signals comprising a plurality of ultrasound signals to an object in different directions;
   transmitting a detection ultrasound signal to the object in which a shear wave is generated by the unfocused push signals;
   receiving a response signal in response to the detection ultrasound signal from the object; and
   generating the elastography image of the object based on the response signal.

2. The method of claim 1,
   wherein a plurality of transducer elements that transmit the unfocused push signals are classified into a first group and a second group comprising a same number of adjacent transducer elements with respect to a center of an array in which the plurality of transducer elements are arranged,
   wherein the unfocused push signals comprise a first unfocused push signal transmitted from the first group and a second unfocused push signal transmitted from the second group, and
   the first unfocused push signal and the second unfocused push signal are transmitted in different directions such that the first unfocused push signal and the second unfocused push signal are symmetrical to each other with respect to a predetermined axis, and the predetermined axis is parallel to an axial direction of the plurality of transducer elements at the center of the array.

3. An ultrasound diagnosis apparatus comprising:

   a transmission unit configured to transmit unfocused push signals comprising a plurality of ultrasound signals to an object in different directions and to transmit a detection ultrasound signal to the object in which a shear wave is generated by the unfocused push signals;
   an ultrasound transmission/reception unit comprising a reception unit configured to receive a response signal in response to the detection ultrasound signal from the object; and
   an image processing unit configured to generate an elastography image of the object based on the response signal.

4. The ultrasound diagnosis apparatus of claim 3, wherein the unfocused push signals comprise the plurality of ultrasound signals transmitted to a plurality of focal points inside the object and generate the shear wave inside the object by an acoustic radiation force generated at the plurality of focal points.

5. The ultrasound diagnosis apparatus of claim 3, wherein the transmission unit transmits the unfocused push signals to the object in different directions such that the unfocused push signals simultaneously arrive at a predetermined axis.

6. The ultrasound diagnosis apparatus of claim 5, wherein the unfocused push signals are transmitted in different directions so that constructive interference occurs between the unfocused push signals on the predetermined axis, and the predetermined axis is parallel to an axial direction of a plurality of transducer elements that transmit the unfocused push signals.

7. The ultrasound diagnosis apparatus of claim 3, wherein the transmission unit transmits a first unfocused push signal transmitted from a first group of adjacent transducer elements included in a plurality of transducer elements and a second unfocused push signal transmitted from a second group of adjacent transducer elements included in the plurality of transducer elements, and
   the first unfocused push signal and the second unfocused push signal are transmitted in different directions such that the first unfocused push signal and the second unfocused push signal are symmetrical to each other with respect to a predetermined axis.

**8.** The ultrasound diagnosis apparatus of claim 3,

wherein a plurality of transducer elements that transmit the unfocused push signals are classified into a first group and a second group comprising a same number of adjacent transducer elements with respect to a center of an array in which the plurality of transducer elements are arranged,

wherein the unfocused push signals comprise a first unfocused push signal transmitted from the first group and a second unfocused push signal transmitted from the second group, and

the first unfocused push signal and the second unfocused push signal are transmitted in different directions such that the first unfocused push signal and the second unfocused push signal are symmetrical to each other with respect to a predetermined axis, and the predetermined axis is parallel to an axial direction of the plurality of transducer elements at the center of the array.

**9.** The ultrasound diagnosis apparatus of claim 8, wherein the transmission unit transmits the first unfocused push signal in a direction inclined by $+\theta°$ with respect to the predetermined axis and transmits the second unfocused push signal in a direction inclined by $-\theta°$ with respect to the predetermined axis.

**10.** The ultrasound diagnosis apparatus of claim 3,

wherein the unfocused push signals comprise the plurality of ultrasound signals transmitted from the respective plurality of transducer elements,

wherein the plurality of transducer elements transmit the plurality of ultrasound signals whose intensity is reduced from a transducer element located at a center of an array in which the plurality of transducer elements are arranged to a transducer element located at an end of the array.

**11.** An ultrasound diagnosis apparatus comprising:

a transmission unit configured to transmit a first push signal comprising one or more ultrasound signals to an object, to transmit a second push signal comprising one or more ultrasound signals to the object based on information regarding propagation of a first shear wave generated inside the object by the first push signal, and to transmit a detection ultrasound signal to the object in which the first shear wave is present and a second shear wave is generated by the second push signal;

an ultrasound transmission/reception unit comprising a reception unit configured to receive a response signal in response to the detection ultrasound signal from the object; and

an image processing unit configured to generate an elastography image of the object based on the response signal.

**12.** An ultrasound diagnosis apparatus comprising:

a transmission unit configured to transmit a push signal comprising a plurality of ultrasound signals to an object, to stop sequentially transmitting of the plurality of ultrasound signals in a first direction, and to transmit a detection ultrasound signal to the object in which a shear wave is generated by transmitting the plurality of ultrasound signals;

an ultrasound transmission/reception unit comprising a reception unit configured to receive a response signal in response to the detection ultrasound signal from the object; and

an image processing unit configured to generate an elastography image of the object based on the response signal.

**13.** An ultrasound diagnosis apparatus comprising:

a transmission unit configured to transmit repeatedly a push signal comprising one or more ultrasound signals to an object, to transmit a first detection ultrasound signal having a first time offset by transmitting the push signal to the object in which a shear wave is generated by the push signal, and to transmit a second detection ultrasound signal having a second time offset by retransmitting the push signal to the object in which a shear wave is generated by retransmitting the push signal;

an ultrasound transmission/reception unit comprising a reception unit configured to acquire first ultrasound image data from a first response signal in response to the first detection ultrasound signal and to acquire second ultrasound image data from a second response signal in response to the second detection ultrasound signal; and

an image processing unit configured to combine the first ultrasound image data and the second ultrasound image data based on the first time offset and the second time offset and to generate an elastography image of the object based on the combined ultrasound image data.

**14.** An ultrasound diagnosis apparatus comprising:

a transmission unit configured to transmit a push signal comprising one or more ultrasound signals to an object, to transmit a first detection ultrasound signal focused on a first scan line among a plurality of scan lines included in the object in which a shear wave is generated by the push signal, and to transmit a second detection ultrasound signal focused on a second scan line among the plurality of scan lines;
an ultrasound transmission/reception unit comprising a reception unit configured to receive a first response signal in response to the first detection ultrasound signal from the object and to receive a second response signal in response to the second detection ultrasound signal from the object; and
an image processing unit configured to generate an elastography image of the object by using the first response signal and the second response signal, to extract first image data corresponding to the first scan line from the first response signal, and to extract second image data corresponding to the second scan line from the second response signal, to combine the first image data and the second image data, and to generate a B mode image.

**15.** A non-transitory computer-readable recording medium having recorded thereon a program for executing the method of generating an elastography image of claim 1.

# FIG. 1

# FIG. 2

# FIG. 3

1000

1050

MAIN BODY

1020

ULTRASOUND
TRANSMISSION
/ RECEPTION UNIT

1021

TRANSMISSION
UNIT

1022

RECEPTION
UNIT

1010

PROBE

1030

IMAGE
PROCESSING
UNIT

FIG. 4

Intensity  N = 12, f$_c$ = 4.09 MHz                    410

Axial Profile                    420

# FIG. 5

510

Intensity  N = 24, $f_c$ = 4.09 MHz

520

Axial Profile

# FIG. 6

START

TRANSMIT UNFOCUSED PUSH SIGNALS INCLUDING PLURALITY OF ULTRASOUND SIGNALS TO OBJECT IN DIFFERENT DIRECTIONS — S610

TRANSMIT DETECTION ULTRASOUND SIGNAL TO OBJECT IN WHICH SHEAR WAVE IS GENERATED BY UNFOCUSED PUSH SIGNALS — S620

RECEIVE RESPONSE SIGNAL TO DETECTION ULTRASOUND SIGNAL FROM OBJECT — S630

GENERATE ELASTOGRAPHY IMAGE OF OBJECT BASED ON RESPONSE SIGNAL — S640

END

# FIG. 7

FIG. 8

FIG. 9

910

920

Axial Profile

# FIG. 10

START

TRANSMIT FIRST PUSH SIGNAL INCLUDING AT
LEAST ONE ULTRASOUND SIGNAL TO OBJECT — S101

TRANSMIT SECOND PUSH SIGNAL INCLUDING
AT LEAST ONE ULTRASOUND SIGNAL TO OBJECT
BASED ON INFORMATION REGARDING FIRST
SHEAR WAVE GENERATED BY FIRST PUSH SIGNAL
INSIDE OBJECT — S102

TRANSMIT DETECTION ULTRASOUND SIGNAL TO
OBJECT IN WHICH FIRST SHEAR WAVE IS PRESENT
AND SECOND SHEAR WAVE IS GENERATED BY
SECOND PUSH SIGNAL AND RECEIVE RESPONSE
SIGNAL TO DETECTION ULTRASOUND SIGNAL
FROM OBJECT — S103

GENERATE ELASTOGRAPHY IMAGE OF
OBJECT BASED ON RESPONSE SIGNAL — S104

END

FIG. 11

LATERAL
DIRECTION

AXIAL
DIRECTION

115 {

1    2    3    4    5

110

111
113    112
114

FIG. 12

# FIG. 13

START

TRANSMIT PUSH SIGNAL INCLUDING
PLURALITY OF ULTRASOUND SIGNALS
TO OBJECT — S131

SEQUENTIALLY STOP TRANSMITTING
ULTRASOUND SIGNALS IN PREDETERMINED
DIRECTION — S132

TRANSMIT DETECTION ULTRASOUND SIGNAL
TO OBJECT IN WHICH SHEAR WAVE IS
GENERATED BY TRANSMISSION OF
ULTRASOUND SIGNALS AND RECEIVE
RESPONSE SIGNAL TO DETECTION
ULTRASOUND SIGNAL FROM OBJECT — S133

GENERATE ELASTOGRAPHY IMAGE OF
OBJECT BASED ON RESPONSE SIGNAL — S134

END

FIG. 14

145

x

t

ULTRASOUND
SIGNAL on

t₁
t₂
t₃
t₄
t₅
t₆

140

ULTRASOUND
SIGNAL off

141-1    141-3    141-5
    141-2    141-4    141-6

FIG. 15

FIG. 16

# FIG. 17

START

TRANSMIT PUSH SIGNAL INCLUDING AT LEAST ONE ULTRASOUND SIGNAL TO OBJECT — S171

TRANSMIT FIRST DETECTION ULTRASOUND SIGNAL HAVING FIRST TIME OFFSET TO OBJECT IN WHICH SHEAR WAVE IS GENERATED BY PUSH SIGNAL BY TRANSMITTING PUSH SIGNAL AND ACQUIRE FIRST ULTRASOUND IMAGE DATA FROM FIRST RESPONSE SIGNAL TO FIRST DETECTION ULTRASOUND SIGNAL — S172

RETRANSMIT PUSH SIGNAL TO OBJECT — S173

TRANSMIT SECOND DETECTION ULTRASOUND SIGNAL HAVING SECOND TIME OFFSET TO OBJECT IN WHICH SHEAR WAVE IS GENERATED BY RETRANSMITTED PUSH SIGNAL BY RETRANSMITTING PUSH SIGNAL AND ACQUIRE SECOND ULTRASOUND IMAGE DATA FROM SECOND RESPONSE SIGNAL TO SECOND DETECTION ULTRASOUND SIGNAL — S174

COMBINE FIRST ULTRASOUND IMAGE DATA AND SECOND ULTRASOUND IMAGE DATA BASED ON FIRST TIME OFFSET AND SECOND TIME OFFSET — S175

GENERATE ELASTOGRAPHY IMAGE OF OBJECT USING COMBINED ULTRASOUND IMAGE DATA — S176

END

FIG. 18

SEQUENCE 1    185    181-1        181-2        181-3                    DETECTION
                                                                        ULTRASOUND SIGNAL

181 ───▶    PUSH
            SIGNAL                                              • • •

                    1            11           21                    81           91        TIME

SEQUENCE 2    186    182-1        182-2        182-3                    DETECTION
                                                                        ULTRASOUND SIGNAL

182 ───▶    PUSH
            SIGNAL                                              • • •

                    2            12           22                    82           92        TIME

SEQUENCE 10   187    183-1        183-2        183-3                    DETECTION
                                                                        ULTRASOUND SIGNAL

183 ───▶    PUSH
            SIGNAL                                              • • •

                    10           20           30                    90           100       TIME

FIG. 19

## FIG. 20

FIG. 21

0s~1/1000s 211

| 1 | 2 | 3 | ⋯ | 9 | 10 |

1/1000s~2/1000s 212

| 1 | 2 | 3 | ⋯ | 9 | 10 |

⋯

999/1000s~1s 213

| 1 | 2 | 3 | ⋯ | 9 | 10 |

FIG. 22

FIG. 23

LATERAL
DIRECTION

AXIAL
DIRECTION

# FIG. 24

START

TRANSMIT PUSH SIGNAL INCLUDING AT LEAST ONE ULTRASOUND SIGNAL TO OBJECT ── S241

TRANSMIT FIRST DETECTION ULTRASOUND SIGNAL FOCUSED ON FIRST SCAN LINE AMONG PLURALITY OF SCAN LINES INCLUDED IN OBJECT IN WHICH SHEAR WAVE IS GENERATED BY PUSH SIGNAL AND RECEIVE FIRST RESPONSE SIGNAL TO FIRST DETECTION ULTRASOUND SIGNAL FROM OBJECT ── S242

TRANSMIT SECOND DETECTION ULTRASOUND SIGNAL FOCUSED ON SECOND SCAN LINE AMONG PLURALITY OF SCAN LINES AND RECEIVE SECOND RESPONSE SIGNAL TO SECOND DETECTION ULTRASOUND SIGNAL FROM OBJECT ── S243

GENERATE ELASTOGRAPHY IMAGE USING FIRST AND SECOND RESPONSE SIGNALS ── S244

EXTRACT FIRST IMAGE DATA CORRESPONDING TO FIRST SCAN LINE FROM FIRST RESPONSE SIGNAL, EXTRACT SECOND IMAGE DATA CORRESPONDING TO SECOND SCAN LINE FROM SECOND RESPONSE SIGNAL AND GENERATE IMAGE INDICATING OBJECT BY COMBINING FIRST AND SECOND IMAGE DATA ── S245

END

FIG. 25

FIG. 26

FIG. 27

## INTERNATIONAL SEARCH REPORT

| International application No. |
|---|
| **PCT/KR2014/007326** |

| A. CLASSIFICATION OF SUBJECT MATTER |
|---|
| *A61B 8/00(2006.01)i* |
| According to International Patent Classification (IPC) or to both national classification and IPC |

| B. FIELDS SEARCHED |
|---|
| Minimum documentation searched (classification system followed by classification symbols) |
| A61B 8/00; G01N 9/24; A61B 8/08; G01N 21/00; G06T 7/20; G01N 29/00 |
| Documentation searched other than minimum documentation to the extent that such documents are included in the fields searched<br>Korean Utility models and applications for Utility models: IPC as above<br>Japanese Utility models and applications for Utility models: IPC as above |
| Electronic data base consulted during the international search (name of data base and, where practicable, search terms used)<br>eKOMPASS (KIPO internal) & Keywords: unfocusing, push signal, shear wave, elastography, acoustic radiation force, pressure |

| C. DOCUMENTS CONSIDERED TO BE RELEVANT | | |
|---|---|---|
| Category* | Citation of document, with indication, where appropriate, of the relevant passages | Relevant to claim No. |
| Y | WO 2012-116364 A1 (MAYO FOUNDATION FOR MEDICAL EDUCATION AND RESEARCH) 30 August 2012<br>See abstract, paragraphs [0014], [0015], [0052]-[0073], claim 1 and figures 1-13. | 1,3,4,10,15 |
| A | | 2,5-9,11-14 |
| Y | KR 10-2012-0039545 A (SUPER SONIC IMAGINE et al.) 25 April 2012<br>See abstract, paragraphs [0011], [0030], [0078]-[0084] and figure 1. | 1,3,4,10,15 |
| A | JP 2013-523323 A (HITACHI ALOKA MEDICAL LTD.) 17 June 2013<br>See abstract, paragraphs [0025]-[0047] and figures 1-13. | 1-15 |
| A | US 2012-0158323 A1 (HAZARD, Christopher Robert et al.) 21 June 2012<br>See abstract, paragraphs [0017]-[0039] and figures 1-4. | 1-15 |
| A | US 2012-0123262 A1 (XIE, Hua et al.) 17 May 2012<br>See abstract, paragraphs [0044]-[0089] and figures 1-8. | 1-15 |

| ☐ Further documents are listed in the continuation of Box C. | ☒ See patent family annex. |
|---|---|

| * Special categories of cited documents: | "T" later document published after the international filing date or priority date and not in conflict with the application but cited to understand the principle or theory underlying the invention |
|---|---|
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
|---|---|
| 21 NOVEMBER 2014 (21.11.2014) | **21 NOVEMBER 2014 (21.11.2014)** |
| Name and mailing address of the ISA/**KR**<br>Korean Intellectual Property Office<br>Government Complex-Daejeon, 189 Seonsa-ro, Daejeon 302-701,<br>Republic of Korea<br>Facsimile No. 82-42-472-7140 | Authorized officer<br><br>Telephone No. |

Form PCT/ISA/210 (second sheet) (July 2009)

INTERNATIONAL SEARCH REPORT
Information on patent family members

International application No.

**PCT/KR2014/007326**

| Patent document cited in search report | Publication date | Patent family member | Publication date |
|---|---|---|---|
| WO 2012-116364 A1 | 30/08/2012 | CN 103492855 A | 01/01/2014 |
| | | EP 2678658 A1 | 01/01/2014 |
| | | JP 2014-506523 A | 17/03/2014 |
| | | KR 10-2014-0034161 A | 19/03/2014 |
| | | US 2014-0046173 A1 | 13/02/2014 |
| KR 10-2012-0039545 A | 25/04/2012 | CA 2764263 A1 | 09/12/2010 |
| | | CN 102458260 A | 16/05/2012 |
| | | EP 2437666 A1 | 11/04/2012 |
| | | EP 2437666 B1 | 01/05/2013 |
| | | JP 2012-528614 A | 15/11/2012 |
| | | JP 5594361 B2 | 24/09/2014 |
| | | US 2010-0312116 A1 | 09/12/2010 |
| | | WO 2010-139519 A1 | 09/12/2010 |
| JP 2013-523323 A | 17/06/2013 | CN 102821700 A | 12/12/2012 |
| | | CN 102892358 A | 23/01/2013 |
| | | CN 103096812 A | 08/05/2013 |
| | | EP 2555684 A2 | 13/02/2013 |
| | | EP 2555684 A4 | 20/08/2014 |
| | | EP 2555685 A2 | 13/02/2013 |
| | | EP 2555685 A4 | 20/08/2014 |
| | | EP 2555686 A2 | 13/02/2013 |
| | | EP 2555686 A4 | 20/08/2014 |
| | | JP 2013-523324 A | 17/06/2013 |
| | | JP 2013-523325 A | 17/06/2013 |
| | | US 2011-0245668 A1 | 06/10/2011 |
| | | US 2011-0245672 A1 | 06/10/2011 |
| | | US 2011-0245678 A1 | 06/10/2011 |
| | | US 8715185 B2 | 06/05/2014 |
| | | WO 2011-126727 A2 | 13/10/2011 |
| | | WO 2011-126727 A3 | 29/12/2011 |
| | | WO 2011-126728 A2 | 13/10/2011 |
| | | WO 2011-126728 A3 | 29/12/2011 |
| | | WO 2011-126729 A2 | 13/10/2011 |
| | | WO 2011-126729 A3 | 29/12/2011 |
| US 2012-0158323 A1 | 21/06/2012 | DE 102011054467 A1 | 21/06/2012 |
| | | FR 2969308 A1 | 22/06/2012 |
| | | JP 2012-125549 A | 05/07/2012 |
| | | US 8494791 B2 | 23/07/2013 |
| US 2012-0123262 A1 | 17/05/2012 | CN 102481137 A | 30/05/2012 |
| | | EP 2448494 A1 | 09/05/2012 |
| | | JP 2012-531937 A | 13/12/2012 |
| | | RU 2012103016 A | 10/08/2013 |
| | | US 2012-123492 A1 | 17/05/2012 |
| | | WO 2011-001333 A1 | 06/01/2011 |

Form PCT/ISA/210 (patent family annex) (July 2009)